# FASCICULE DE BREVET EUROPEEN

(11) **EP 0 805 800 B1**
(45) Date de publication et mention de la délivrance du brevet: **14.03.2001**
(21) Numéro de dépôt: 96938297.7
(22) Date de dépôt: 12.11.1996
(51) Int. Cl.: C07C 317/44, C07C 317/46, C07D 319/06, C07D 309/30

(54) **PROCEDE DE PREPARATION DE COMPOSES A GROUPEMENT BETA-HYDROXY-DELTA-LACTONE ANALOGUES DE LA (+) COMPACTINE ET DE LA (+) MEVINOLINE**
VERFAHREN ZUR HERSTELLUNG VON (+)COMPACTIN UND (+)MEVINOLIN DERIVATEN MIT BETA-HYDROXY-DELTA-LACTONRESTEN
METHOD FOR PREPARING (+)COMPACTIN AND (+)MEVINOLIN ANALOG COMPOUNDS HAVING A BETA-HYDROXY-DELTA-LACTONE GROUPING

(30) Priorité: 28.11.1995 FR 9514093
(43) Date de publication de la demande: 12.11.1997
(73) Titulaire: L'OREAL, 75008 Paris (FR)
(72) Inventeur: SOLLADIE, Guy, F-67000 Strasbourg (FR); BAUDER, Claude, F-67000 Strasbourg (FR)
(74) Mandataire: Tezier Herman, Béatrice
(86) Numéro de dépôt international: FR9601782
(87) Numéro de publication internationale: WO9719917

(56) Documents cités:
- TETRAHEDRON: ASYMMETRY, vol. 6, no. 11, Novembre 1995, OXFORD, GB, pages 2679-2682, XP002024874 G. SOLLADIE, ET AL.: "Enatioselective synthesis of a bicyclic ketal induced by chiral sulphoxides: (-)-(1R,3R,5S)-endo- 1,3-dimethyl-2,9-dioxabicyclo[3.3.1]nonane "
- JOURNAL OF ORGANIC CHEMISTRY, vol. 60, no. 24, 1 Décembre 1995, WASHINGTON, DC, US, pages 7774-7777, XP002024875 G. SOLLADIE, ET AL.: "Chiral sulphoxides in asymmetric synthesis: enantioselective synthesis of the lactonic moiety of (+)-compactin and (+)-mevinolin. Application to a compactin analogue"
- TETRAHEDRON LETTERS, vol. 33, no. 12, 17 Mars 1992, OXFORD, GB, pages 1605-1608, XP002010057 G. SOLLADIE, ET AL.: "Asymmetric synthesis of beta-hydroxyketones, precursors of chiral 1,3-diols, from beta,delta-diketosulphoxides"
- TETRAHEDRON: ASYMMETRY, vol. 3, no. 1, Janvier 1992, OXFORD, GB, pages 33-38, XP002010058 G. SOLLADIE, ET AL.: "Synthesis of optically active beta,delta-diketo-p-tolylsulphoxides"
- TETRAHEDRON: ASYMMETRY, vol. 5, no. 9, Septembre 1994, OXFORD, GB, pages 1717-1726, XP002010059 G. SOLLADIE, ET AL.: "Asymmetric synthesis induced by chiral sulphoxides: 2-deoxy-sugars from beta-ketoesters via malic acid"
- TETRAHEDRON LETTERS, vol. 31, no. 18, 1990, OXFORD, GB, pages 2545-2548, XP002010060 G. WESS, ET AL.: "Stereoselective synthesis of HR 780 a new highly potent HMG-CoA reductase inhibitor"
- TETRAHEDRON: ASYMMETRY, vol. 1, no. 5, 1990, OXFORD, GB, pages 307-310, XP000142279 K. PRASAD, ET AL.: "A highly stereoselective route to the four stereoisomers of a six-carbon synthon"
- TETRAHEDRON LETTERS, vol. 25, no. 23, 1984, OXFORD, GB, pages 2435-2438, XP002010062 K. PRASAD, ET AL.: "A novel diastereoselective synthesis of the lactone moiety of compactin"

## Description

L'invention a pour objet un nouveau procédé de préparation de composés à groupement β-hydroxy-δ-lactone analogues de la (+)Compactine et de la (+)Mevinoline. Le procédé de préparation de ces produits fait intervenir de nouveaux intermédiaires réactionnels, également objets de l'invention ainsi que leurs procédés de préparation respectifs.

Les inhibiteurs d'HMG - Coenzyme A-réductase (β-hydroxy-β-méthyl Glutaryl - Coenzyme A-réductase notée ci-après HMG-CoA-réductase), famille à laquelle appartiennent la (+)Compactine et la (+)Mevinoline, sont des actifs pharmaceutiques utilisés par voie orale dans le traitement des hypercholestérolémies (taux plasmatiques de cholestérol trop élevés). Voir à cet effet le document « Pharmacologie, Des concepts fondamentaux aux applications thérapeutiques », publié sous la direction de M. Schorderet, Editions Frison-Roche, seconde édition, 1992.

Ces inhibiteurs sont également utilisés dans le traitement des maladies liées à la présence excessive de cholestérol, telles que l'artériosclérose.

La (+) Compactine (formule **1** ci-après avec R=H) et la (+) Mevinoline (formule **1** avec R=CH₃) sont des molécules bien connues pour leur activité d'inhibiteur d'HMG-CoA-réductase :

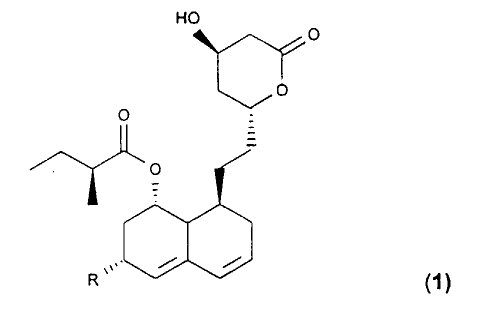

En outre, de nombreux travaux ont été développés autour du fragment lactonique de ces molécules. En particulier on trouve dans les documents Chapleur, Y. « Progress in the chemical synthesis of antibiotics and related microbial products », Springer Verlag 1993, vol.2, 829-937 (Doc I), et T. Rosen et C.H. Heathcock Tetrahedron, Vol.42, N°18, 4909-4951, 1986 (Doc II), une revue des synthèses d'analogues de la (+)Compactine et de la (+)Mevinoline, analogues ayant conservé le fragment lactonique de ces dernières et dont la partie lipophile est simplifiée par rapport à la (+)-Compactine et à la (+)-Mevinoline. Toutefois, aucune des voies de synthèse connues pour de telles molécules n'est satisfaisante.

Les principales voies de synthèse du fragment lactonique de la lactone **1**, sous forme optiquement active, utilisent des produits de départ commerciaux chiraux comme l'acide malique ou l'acide 2-glutamique (M. Majewski & co Tet.Lett. 1984, 25, 2101 ; T. Minami & co Tet.Lett. 1993, 34, 513) ou des carbohydrates (M. Menges & co Synlett 1993, 901 ; M.S. Ernolenko & co, Tet.Lett.1994, 35, 715). D'autres comprennent une étape de réduction asymétrique avec des catalyseurs au Rhodium (M. Terada & co, Tet.Lett.1991, 32, 935 ; L. Shao & co, Tet.Lett.1991, 32, 7699) ou des réducteurs enzymatiques (F. Bennet & co, J.Chem.Soc. Perkin Transfer I, 1991, 133 ; M.H. Ansari, Tet.Lett. 1993, 34, 8271).

Ces synthèses ne sont pas dénuées d'inconvénients. En particulier, les produits de départ chiraux employés dans certaines de ces synthèses sont couteux, de même que sont très couteux les catalyseurs permettant d'induire une réduction asymétrique. D'autre part, les réductions asymétriques par voie enzymatique donnent des excès énantiomériques qui ne dépassent pas 80%.

On connait également des synthèses qui ne présentent pas ces inconvénients comme celle décrite dans Tet.Lett., Vol.35, N°5, 715-718, 1994 mais nécéssitent d'utiliser des réactifs et solvants dangereux, comme la pyridine et le tri-butylétain.

Aussi, c'est avec étonnement que la demanderesse a découvert une nouvelle voie de synthèse d'analogues de la (+) Compactine et de la (+) Mevinoline. Cette voie de synthèse, facile à mettre en oeuvre, extrapolable à l'échelle industrielle, utilise un produit de départ peu couteux, un agent chiral qui peut être fabriqué industriellement et présente un coût modéré. De plus, elle offre de bons rendements, et les produits de cette synthèse sont obtenus avec une excellente pureté énantiomérique (>98%). En outre, cette voie de synthèse permet d'accéder à de nouveaux analogues de la (+) Compactine et de la (+) Mevinoline.

L'invention a pour objet un nouveau procédé de préparation des produits répondant à l'une des formules (la) et (Ib) : 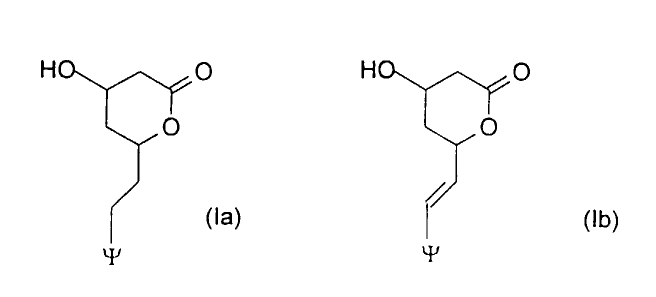 dans lesquelles Ψ représente un radical choisi parmi :
- les alkyles en C1-C40, linéaires ramifiés ou cycliques, saturés ou insaturés, éventuellement interrompus par des ponts éthers et/ou éventuellement substitués par un ou plusieurs substituants choisis parmi les groupements halogène, hydroxyle, alcoxy en C1-C20, acyloxy en C1-C20, amino primaire secondaire ou tertiaire, nitro, thiol, carboxyle, amido, carboxylate d'alkyle en C1-C20, carboxylate d'aryle, carboxylate d'aralkyle en C1-C20, alkylamido en C1-C20, arylamido, aralkylamido en C1-C20, et parmi les radicaux aryle et les hétérocycles azotés, oxygénés, phosphorés et soufrés en C1-C20, saturés ou insaturés.
- les aryles, éventuellement substitués par un ou plusieurs substituants choisis parmi les groupements halogène, hydroxyle, alcoxy en C1-C20, acyloxy en C1-C20, amino alkylamino et dialkylamino en C1-C20, nitro, thiol, carboxyle, amido, carboxylate d'alkyle en C1-C20, carboxylate d'aryle, carboxylate d'aralkyle en C1-C20, alkylamido en C1-C20, arylamido, aralkylamido en C1-C20, aryles en C1-C20 et parmi les radicaux alkyles et aralkyles en C1-C20, saturés ou insaturés, linéaires, ramifiés ou cycliques, les hétérocycles azotés, oxygénés, phosphorés et soufrés en C1-C20, saturés ou insaturés.
- les aralkyles, éventuellement substitués par un ou plusieurs substituants choisis parmi les groupements halogène, hydroxyle, alcoxy en C1-C20, acyloxy en C1-C20, amino alkylamino et dialkylamino en C1-C20, nitro, thiol, acide carboxylique , amido, carboxylate d'alkyle en C1-C20, carboxylate d'aryle, carboxylate d'aralkyle en C1-C20, alkylamido en C1-C20, arylamido, aralkylamido en C1-C20, aryles en C1-C20 et parmi les radicaux alkyles et aralkyles en C1-C20, saturés ou insaturés, linéaires, ramifiés ou cycliques.
- les hétérocycles azotés, oxygénés phosphorés ou soufrés, éventuellement substitués par un ou plusieurs substituants choisis parmi les groupements halogène, hydroxyle, alcoxy en C1-C20, acyloxy en C1-C20, amino primaire secondaire ou tertiaire, nitro, thiol, acide carboxylique , amido, carboxylate d'alkyle en C1-C20, carboxylate d'aryle, carboxylate d'aralkyle en C1-C20, alkylamido en C1-C20, arylamido, aralkylamido en C1-C20, et parmi les radicaux aryles, aralkyles, les hétérocycles azotés, oxygénés et soufrés en C1-C20, saturés ou insaturés.

Les formules (la) et (Ib) couvrent un nombre de produits très important, dont l'étendue dans le domaine des inhibiteurs de HMG - Coenzyme A-réductase est illustrée par les documents « Progress in the chemical synthesis of Antibiotics and related microbial products » Y. Chapleur, vol.2, 1993, 829-937, G.Lukacs Ed. Springer Verlag (Doc I) ; T. Rosen et C.H. Heathcock Tetrahedron, Vol.42, N°18, 4909-4951, 1986 (Doc II) et J. Prous « The year's drug news, therapeutic targets, 1994 edition », Prous science Publishers (Doc III). Outre les produits mentionnés dans ces documents, le procédé objet de la présente invention permet un accès facile à de nombreuses autres molécules répondant aux formules la et lb.

Parmi les radicaux représentés par Ψ, on peut citer par exemple les radicaux répondant au formules suivantes et qui correspondent aux produits de formule (la) connus comme inhibiteurs de HMG - Coenzyme A-réductase :

Décrits dans Doc I :

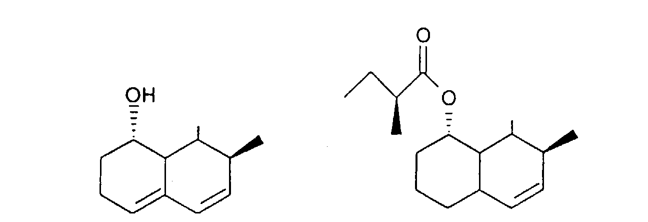

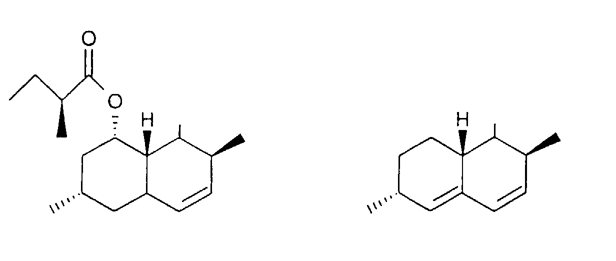

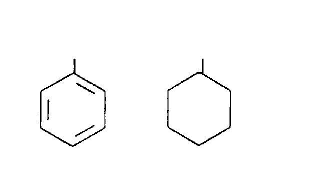

Décrits dans Doc III :

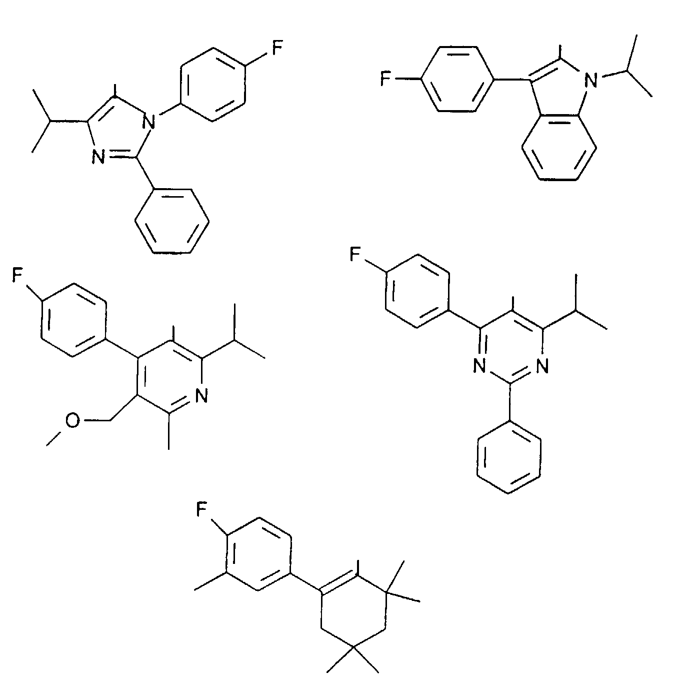

Les produits de formule (la) et de formule (Ib) comportent au moins deux carbones asymétriques. Aussi l'invention a pour objet un procédé de préparation de chacun des quatre diastéréoisomères répondant à l'une des formules (la) ou (Ib) pris isolément, ainsi que de leurs mélanges racémiques ou non.

L'invention a pour objet un procédé de préparation des produits de formule (la) et (Ib), caractérisé en ce que :
1) dans une première étape, on fait réagir le tri-anion du 3,5-di-oxo-hexanoate d'alkyle de formule (X) représentée ci-dessous, avec le (X's)-p-toluène (noté pTol) sulfinate de (-) ou de (+) menthyle pour obtenir le (Xs)-3,5-di-oxo-6-p-tolylsulfinyl hexanoate d'alkyle de formule (II),

   Y-CO-CH₂-CO-CH₂-CO-CH₃ (X)

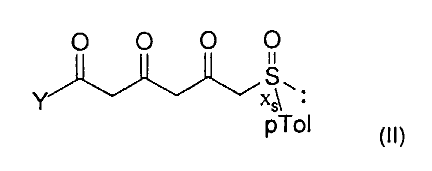

   Xs et X's désignant les configurations *R* ou *S* du soufre dans les deux molécules, avec Xs ≠ X's, Y représentant un radical alkyloxy en C1-C18 linéaire ou ramifié, saturé ou insaturé, un radical aryloxy ou un radical aralkyloxy en C1-C18, un radical imidazolide, un radical alkylsulfure en C1-C18 linéaire ou ramifié, saturé ou insaturé, arylsulfure, aralkylsulfure en C1-C18, un radical 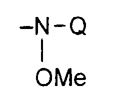 Q désignant un radical alkyle en C1-C18 linéaire ou ramifié, saturé ou insaturé Quel que soit le choix de Y on désignera le groupement -COY du nom d'ester.
2) dans une seconde étape, on réduit de façon énantiosélective la fonction 5-carbonyle du (Xs)-3,5-di-oxo-6-paratolylsulfinyl hexanoate d'alkyle en alcool pour donner le [5(X₅), S(Xs)]-5-hydroxy-3-oxo-6-paratolylsulfinyl-hexanoate d'alkyle de formule (III) ci-après, X₅ désignant la configuration du carbone C₅, avec X₅ ≠ Xs et Y ayant la même signification que ci-dessus, 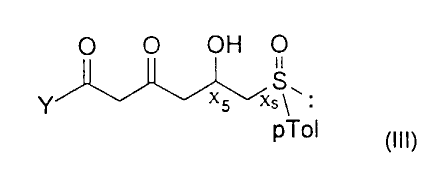
3) dans une troisième étape, on réduit de façon diastéréosélective la fonction 3-carbonyle du [5(X₅), S(Xs)]-5-hydroxy-3-oxo-6-paratolylsulfinyl-hexanoate d'alkyle pour obtenir le [3 (X₃), 5(X₅), S(Xs)]-3,5-dihydroxy-6-paratolylsulfinyl-hexanoate d'alkyle de formule (IV) ci-après, X₃ désignant la configuration du carbone C₃, en choisissant une méthode de réduction donnant X₅ = X₃ si l'on veut une configuration anti ou une méthode de réduction donnant X₅ ≠ X₃ si l'on veut une configuration syn des deux fonctions alcool, Y ayant la même signification que ci-dessus, 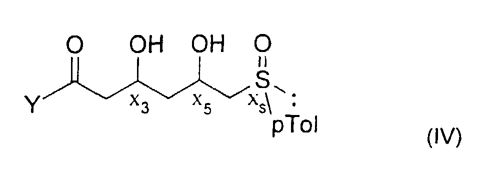
4) dans une quatrième étape, on protège les deux fonctions hydroxyle par des radicaux alkyle en C1-C8 ou aryle ou aralkyle, des radicaux tri-alkylsilyle, ou par un radical alcane di-yle en C1-C8, un radical benzylidène, radicaux que l'on désignera R, pour obtenir un [3(X₃), 5(X₅), S(Xs)]-3,5-di-alkyloxy-6-paratolylsulfinyl-hexanoate d'alkyle de formule (V) ci dessous, Y ayant la même signification que ci-dessus, lesdits radicaux n'étant pas déplacés dans les conditions des étapes 5 et 6, 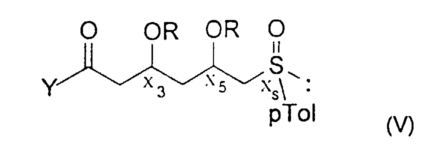
5) dans une cinquième étape, on réduit la fonction sulfoxyde en thioéther par une réaction de Pummerer pour obtenir un [3(X₃), 5(X₅)]-6-acétoxy-3,5-di-alkyloxy-6-paratolyl-thio-hexanoate d'alkyle de formule (VI) ci-après, R et Y ayant la même signification que ci-dessus, 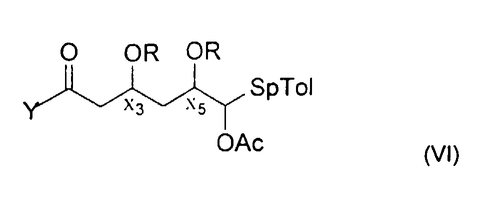
6) dans une sixième étape, on transforme les fonctions en C6 en aldéhyde pour obtenir le [3(X₃), 5(X₅)] 6-oxo 3,5-di-alkyloxy-hexanoate d'alkyle répondant à la formule (VII), Y et R ayant la même signification que ci-dessus, 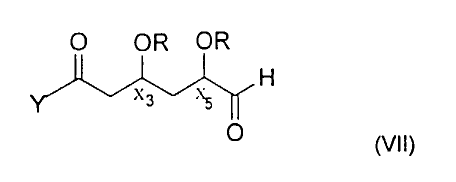
7) dans une septième étape on condense le [3(X₃), 5(X₅)] 6-oxo 3,5-di-alkyloxy-hexanoate d'alkyle de formule (VII) sur un réactif de Wittig Ph₃P=GHΨ, avec Ψ ayant la définition donnée ci-dessus, pour donner le produit de formule (VIII), R et Y ayant la même signification que ci-dessus : 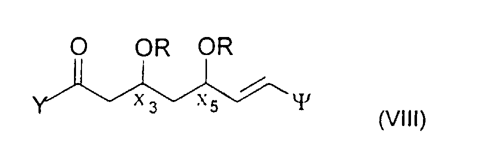
8) dans une huitième étape on réduit la double liaison pour obtenir : 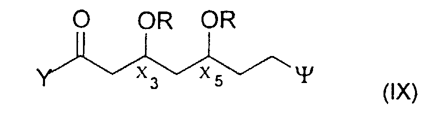
9) dans une neuvième et dernière étape on élimine les groupes protecteurs des hydroxyle et on hydrolyse l'ester pour donner le produit de formule (la).

Selon une variante de l'invention on peut après l'étape 7) passer directement à l'étape 9) sans réduire la double liaison pour obtenir le produit de formule (Ib).

On peut également inverser l'ordre des étapes 8 et 9 pour accéder aux produits de formule (la).

### Etape 1 :

Selon l'invention, on fait réagir le tri-anion du 3,5-dioxohexanoate d'alkyle de formule (X)

Y-CO-CH₂-CO-CH₂-CO-CH₃ (X)

dans laquelle Y a la même définition que ci-dessus, avec le (X's)-p-toluène sulfinate de (-) ou de (+) menthyle pour obtenir le (Xs)-3,5-di-oxo-6-p-tolyl-sulfinyl hexanoate d'alkyle.

De façon préférentielle, on choisira Y parmi les radicaux méthoxy, éthoxy, ter-butyloxy, 1-imidazolidinyle, benzyloxy, N(OMe)Q, avec Q radical alkyle en C1-C6.

En raison de sa plus grande facilité de préparation et de manipulation, on choisit avantageusement Y=OCH₃.

La préparation du 3,5-di-oxo-hexanoate de méthyle, produit de départ, est connue, on pourra par exemple se référer à J.G. Batelan, Synth. Commun. 1976, 6, 81. Cette méthode de préparation peut être facilement appliquée aux autres 3,5-dioxohexanoate d'alkyle.

Pour la préparation des produits de formule (X) avec Y= N(OMe)Q, avec Q radical alkyle en C1-C6, on pourra se référer aux documents suivants : S. Nahm et S.M. Weinreb, Tet. Lett. 1981, Vol.22, 3815-3818 ; T.A. Oster et T.M. Harris, Tet. Lett. 1983, Vol.24, 1851-1854.

Le tri-anion du 3,5-di-oxo-hexanoate d'alkyle est obtenu de préférence par traitement avec de l'hydrure de sodium et soit du ter-butyl lithium soit du sec-butyl lithium en milieu anhydre à basse température. De façon encore plus préférentielle, cette réaction est effectuée en présence d'environ un équivalent d'hydrure de sodium et de deux équivalents de ter-butyl lithium ou en présence d'environ un équivalent d'hydrure de sodium et de deux équivalents de sec-butyl lithium. Préférentiellement la réaction est effectuée dans un solvant choisi parmi les éthers et encore plus préférentiellement le tétrahydrofurane. De façon préférentielle, cette réaction est effectuée à une température allant de -10°C à +10°C. Selon un mode préféré de réalisation de l'invention on fait cette réaction à une température voisine de 0°C.

Le (X's)-p-toluène sulfinate de menthyle, avec X's =*R*,*S*, est connu de l'homme du métier, on pourra par exemple se reporter à G. Solladié, J. Hutt, A. Girardin, Synthesis, 1987, 173. Le choix de l'un ou l'autre des isomères *R* ou *S* du p-toluène sulfinate de menthyle dépend de la configuration que l'on souhaite obtenir sur le carbone C₅ après l'étape 2 de réduction de la cétone en C₅. Selon l'invention, le carbone C₅ aura la même configuration, X₅=X's, que le soufre du (X's)-p-toluène sulfinate de menthyle. De façon préférentielle, cette réaction se fait avec une quantité de p-toluène sulfinate de menthyle sensiblement égale à 50% en nombre de moles par rapport au nombre de moles de 3,5-di-oxo-hexanoate d'alkyle.

On sait que le (+) et le (-) menthol peuvent tous deux conduire à chacun des isomères *R* et *S* du p-toluène sulfinate de menthyle. Toutefois d'après G. Solladié, J. Hutt, A. Girardin, Synthesis, 1987, 173, on connait une méthode de synthèse extrapolable à l'échelle industrielle et permettant de préparer le (*S*) p-toluène sulfinate de (-) menthyle. Cette méthode conduit de façon analogue à partir du (+) menthol au (*R*) p-toluène sulfinate de (+) menthyle. On utilise donc de préférence l'un de ces deux réactifs. De façon préférentielle on souhaite obtenir un produit (la) ou (Ib) ayant la même configuration que les produits naturels connus comme inhibiteurs d'HMG - Coenzyme A-réductase, tels que la (+) Compactine et la (+) Mevinoline. Par conséquent, on utilise préférentiellement dans la première étape le (*S*)-p-toluène sulfinate de menthyle, et pour les raisons exposées ci-dessus on utilise de préférence le (*S*)-p-toluène sulfinate de (-) menthyle.

### Etape 2 :

Selon l'invention, dans la seconde étape, on réduit de façon énantiosélective la fonction 5-carbonyle du (Xs)-3,5-di-oxo-6-paratolylsulfinyl hexanoate d'alkyle en alcool pour donner le [5(X₅), S(Xs)]-5-hydroxy-3-oxo-6-paratolylsulfinyl-hexanoate d'alkyle, avec X₅ ≠ Xs. De façon préférentielle, cette réduction est faite en traitant le (Xs)-3,5-di-oxo-6-p-tolylsulfinyl hexanoate d'alkyle par un hydrure respectant l'induction stéréochimique due au radical (Xs)-paratolylsulfinyle et ne réduisant pas le groupement -COY. En particulier, on peut citer l'hydrure de di-isobutyl aluminium, qui répond à ces conditions. De façon préférentielle cette réaction est conduite en milieu anhydre et les réactifs (produit de formule Il et réducteur) sont introduits à une température inférieure à -20°C. De façon encore plus préférentielle, cette réaction est faite dans le tétrahydrofurane et les réactifs sont introduits à une température de -50°C et encore plus préférentiellement aux environs de -78°C. On laisse ensuite le milieu revenir à température ambiante. Le démarrage de la réaction à basse température a pour but de garantir une stéréochimie exacte de C₅, toutefois cette condition n'est pas impérative et certains dérivés traités à 0°C présenteront la stéréochimie souhaitée.

A l'issue de ce traitement, seul le carbonyle en C₅ a été réduit et il l'a été de façon stéréosélective. Ainsi, la réduction dans les conditions décrites ci-dessus du (*R*)-3,5-di-oxo-6-p-tolylsulfinyl hexanoate de méthyle donne le [5(*S*), S(*R*)]-5-hydroxy-3-oxo-6-paratolylsulfinyl-hexanoate de méthyle et inversement la réduction du (*S*)-3,5-di-oxo-6-p-tolylsulfinyl hexanoate de méthyle donne le [5(*R*), S(*S*)]-5-hydroxy-3-oxo-6-paratolylsulfinyl-hexanoate de méthyle.

### Etape 3 :

Selon l'invention, dans une troisième étape, on réduit de façon diastéréosélective la fonction 3-carbonyle du [5(X₅), S(Xs)]-5-hydroxy-3-oxo-6-paratolylsulfinylhexanoate d'alkyle pour obtenir le [3 (X₃), 5(X₅), S(Xs)]-3,5-dihydroxy-6-paratolylsulfinyl-hexanoate d'alkyle, avec au choix une méthode de réduction donnant X₅ = X₃ si l'on veut une configuration anti ou une méthode de réduction donnant X₅ ≠ X₃ si l'on veut une configuration syn des deux fonctions alcool.

De façon préférentielle, si l'on souhaite obtenir une configuration syn des deux alcools, on fera la réduction en traitant le produit de départ (formule III) par du borohydrure de sodium en présence d'un agent chélatant. On peut citer parmi les agents chélatants les dérivés de métaux comme par exemple les dérivés du zinc du titane et du bore. Par exemple, on peut utiliser pour cette réaction le tétraisopropyloxytitane, le bromure de zinc, le diéthylméthoxyborane. De façon préférentielle on utilisera comme agent chélatant du diéthylméthoxyborane. De façon préférentielle la réaction est conduite en milieu anhydre et les réactifs (produit de formule III, réducteur et agent chélatant) sont introduits à une température inférieure à 0°C. De façon préférentielle, cette réaction est mise en oeuvre en présence d'un excès de diéthylméthoxyborane et de borohydrure de sodium, dans le tétrahydrofurane en introduisant les réactifs à une température inférieure à -20°C et préférentiellement à -78°C.Le démarrage de la réaction à basse température a pour but de garantir une stéréochimie exacte de C₃. Toutefois, cette condition n'est pas impérative et certains dérivés traités à 0°C présenteront la stéréochimie souhaitée.

Si l'on souhaite obtenir une configuration anti des deux alcools en C₃ et C₅, on fera préférentiellement la réduction en traitant le produit de départ (produit de formule III) par du triacétoxyborohydrure de tétraméthylammonium. Pour la mise en oeuvre de cette réaction, on pourra par exemple se référer à G. Solladié, C. Dominguez, J. Org. Chem. 1994, 59, 3898.

Ainsi, par le choix de la configuration *R* ou *S* du p-toluène sulfinate de menthyle et le choix de la méthode de réduction de la troisième étape, on peut parfaitement contrôler la configuration des carbones C₃ et C₅.

### Etape 4 :

Selon l'invention, dans une quatrième étape, on protège les deux fonctions hydroxyle par des radicaux alkyle en C1-C8 ou aryle ou aralkyle des radicaux tri-alkylsilyle, ou par un radical alcane di-yle en C1-C8, un radical benzylidène, radicaux que l'on désignera R. Parmi les radicaux susceptibles d'être employés pour protéger les deux fonctions hydroxyle, on peut citer par exemple les radicaux méthyle, benzyle, terbutyldiméthylsilyle, terbutyldiphénylsilyle, ou les radicaux isopropylidène et benzylidène, ces deux derniers radicaux permettant à eux seuls de protéger simultanément les deux hydroxyle. De façon préférentielle, pour des raisons économiques et de facilité de manipulation on utilise pour cette protection un radical isopropylidène. Cette réaction de protection est faite par des moyens biens connus de l'homme du métier, on pourra par exemple traiter le [3(X₃), 5(X₅), S(Xs)]-3,5-di-hydroxy-6-paratolylsulfinyl-hexanoate d'alkyle par du diméthoxypropane en présence de quantités catalytiques d'acide para toluène sulfonique dans l'acétone.

### Etape 5 :

Selon l'invention, dans une cinquième étape, on réduit la fonction sulfoxyde du produit de formule (V) en thioéther par une réaction de Pummerer pour obtenir un [3(X₃), 5(X₅)]-6-acétoxy-3,5-di-alkyloxy-6-paratolyl-thio-hexanoate d'alkyle. De façon préférentielle, cette réaction est conduite dans l'anhydride acétique ou dans l'anhydride trifluoroacétique, en présence d'un large excès d'acétate de sodium anhydre. Pour la mise en oeuvre de cette réaction on peut par exemple se reporter à Tet. Lett. 23, 5541, 1982.

### Etape 6

Selon l'invention, dans une sixième étape, on transforme les fonctions en C6 du produit de formule (VI) en aldéhyde pour obtenir le [3(X₃), 5(X₅)] 6-oxo 3,5-dialkyloxy-hexanoate d'alkyle répondant à la formule (VII). Ces deux réactions peuvent être réalisées suivant plusieurs variantes :
6.1) Selon une première variante on peut :
   6.1.a) réduire la fonction thioéther et hydrolyser l'acétate pour obtenir un alcool répondant à la formule (XI) : 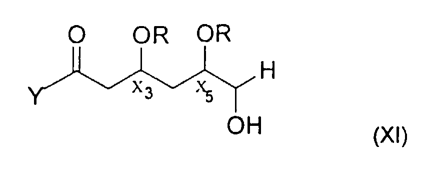 puis :
   6.1.b) oxyder l'alcool en C₆ en aldéhyde. Ces réactions sont bien connues de l'homme du métier, on pourra par exemple éliminer le soufre par un traitement au nickel de Raney dans un solvant protique, comme par exemple le méthanol, ou dans l'éthanol, mais on peut également faire cette réaction dans l'acétate d'éthyle ; puis l'acétate peut être éliminé en traitant le milieu par une base, de préférence du carbonate ou de l'hydrure de di-isobutyl aluminium (DIBAL). De préférence, on élimine le soufre avec un large excès de nickel de Raney, cette quantité étant ajustée en fonction de l'avancement de la réaction.
      L'oxydation de l'hydroxyle en aldéhyde peut être faite par des moyens bien connus de l'homme du métier, comme par exemple par la méthode de Swern, pour laquelle on pourra se référer à Mancuso et Swern, Synthesis, 1981, 165-185.
6.2) Selon une seconde variante, on peut transformer directement les fonctions thioéther et acétate en aldéhyde (VII). Par exemple, on traite le produit (VI) par une base telle qu'un carbonate en milieu aqueux.

### Etape 7 :

Selon l'invention, dans une septième étape on condense l'aldéhyde (VII) sur un réactif de Wittig Ph₃P=CHΨ. Pour la préparation du réactif de Wittig et sa mise en oeuvre, on pourra se reporter à « Advanced Organic Chemistry, Jerry March, 3e édition Wiley, 1985, 845-854 »

### Etape 8 :

La réduction de la double liaison qui constitue la huitième étape du procédé objet de l'invention peut être faite par tout moyen connu de l'homme du métier, comme l'hydrogénation en présence d'un catalyseur, par exemple le palladium sur charbon.

La neuvième étape qui consiste à déprotéger les groupements protecteurs R des hydroxyle fait appel aux connaissance de l'homme du métier. Par exemple un traitement acide en milieu aqueux permet d'hydrolyser les groupements isopropylidène et ester méthylique. Pour la déprotection des produits de formule (VIII) ou (IX) avec Y= N(OMe)Q, avec Q radical alkyle en C1-C6, on pourra se référer aux documents suivants : S. Nahm et S.M. Weinreb, Tet. Lett. 1981, Vol.22, 3815-3818 ; T.A. Oster et T.M. Harris, Tet. Lett. 1983, Vol.24, 1851-1854. Lorsque les fonctions hydroxyle sont protégées par un groupement benzyle ou paranitrobenzyle, on peut avantageusement réduire la double liaison et déprotéger les hydroxyles au cours d'une même étape. De même lorsque l'ester -COY est un ester benzylique, on pourra hydrolyser l'ester en même temps que l'on réduit la double liaison.

L'invention a également pour objet :
- les (Xs)-3,5-dioxo-6-p-tolylsulfinyl hexanoates d'alkyle de formule (II), 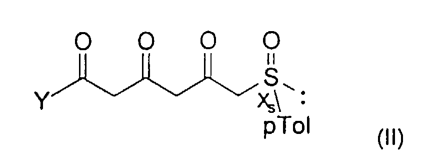 dans laquelle Xs désigne la configuration *R* ou *S* du soufre dans les deux molécules, Y représente un radical alkyloxy en C1-C18 linéaire ou ramifié, saturé ou insaturé, un radical aryloxy ou un radical aralkyloxy en C1-C18, un radical imidazolide, un radical alkylsulfure en C1-C18 linéaire ou ramifié, saturé ou insaturé, arylsulfure ou aralkylsulfure en C1-C18, un radical 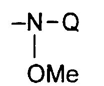 Q désignant un radical alkyle en C1-C18 linéaire ou ramifié, saturé ou insaturé
- les [5(X₅), S(Xs)]-5-hydroxy-3-oxo-6-paratolylsulfinyl-hexanoates d'alkyle de formule (III), 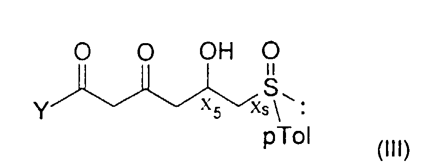 X₅ désignant la configuration du carbone C₅, avec X₅ ≠ Xs, Xs et Y ayant la même signification que ci-dessus,
- les [3(X₃), 5(X₅), S(Xs)]-3,5-dihydroxy-6-paratolylsulfinyl-hexanoates d'alkyle de formule (IV), 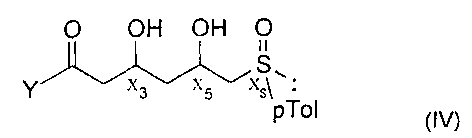 X₃ désignant la configuration *R* ou *S* du carbone C₃, Y , Xs et X₅ ayant la même signification que ci-dessus, avec X₃ = X₅ ou avec X₃ ≠ X₅,
- les [3(X₃), 5(X₅), S(Xs)]-3,5-di-alkyl-oxy-6-paratolylsulfinyl-hexanoates d'alkyle de formule (V), 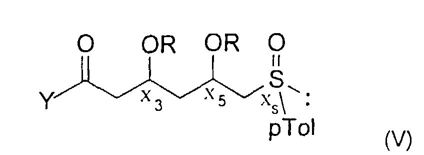 R représentant un radical alkyle en C1-C8 ou un radical aryle ou aralkyle en C1-C8, des radicaux tri-alkylsilyle, ou un radical alcane di-yle en C1-C8, un radical benzylidène, Y, Xs, X₃ et X₅ ayant la même signification que ci-dessus,
- les [3(X₃), 5(X₅)]-6-acétoxy-3,5-di-alkyloxy-6-paratolylthio-hexanoates d'alkyle de formule (VI), 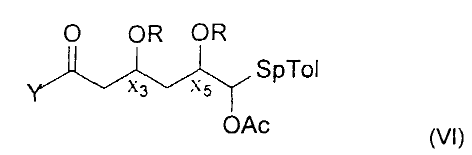 R, Y, X₃ et X₅ ayant la même signification que ci-dessus
- les [3(X₃), 5(X₅)] 6-oxo 3,5-di-alkyloxy-hexanoate d'alkyle répondant à la formule (VII) 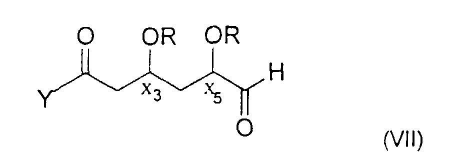 Y, R, X₃ et X₅ ayant la même signification que ci-dessus.
- les [3(X₃), 5(X₅)]-6-acétoxy-3,5-di-alkyloxy-6-hydroxy-hexanoates d'alkyle de formule (XI) 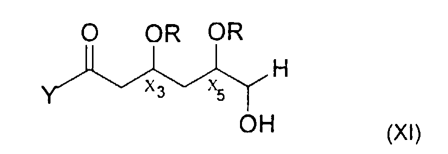 dans laquelle Y représente un radical alkyloxy en C2-C18 linéaire ou ramifié, saturé ou insaturé, un radical aryloxy ou un radical aralkyloxy en C1-C18, un radical imidazolide, un radical alkylsulfure en C1-C18 linéaire ou ramifié, saturé ou insaturé, arylsulfure ou aralkylsulfure en C1-C18, un radical 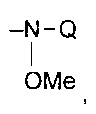 Q désignant un radical alkyle en C1-C18 linéaire ou ramifié, saturé ou insaturé, R, X₃ et X₅ ayant la même signification que ci-dessus.

Selon une variante de l'invention, on peut également préparer les produits de formule (la) par un procédé comprenant les étapes 1 à 5 et 6.1.a, caractérisé en ce que l'on fait le triflate ou le tosylate de l'alcool (XI) puis qu'on le traite par un halogénure HalΨ, Hal désignant un atome de chlore de brome ou d'iode dans des conditions connues de l'homme du métier pour effectuer la substitution qui conduit au produit (IX): 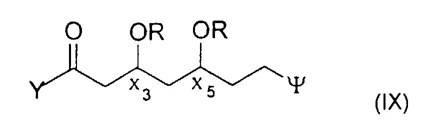 Celui-ci peut ensuite être déprotégé comme décrit ci-dessus pour donner (la).

Plus particulièrement l'invention a pour objet :
- le (*R*)-3,5-dioxo-6-p-tolylsulfinyl hexanoate de méthyle (formule II),
- le [5(*S*), S(*R*)]-5-hydroxy-3-oxo-6-paratolylsulfinyl-hexanoate de méthyle (formule III),
- le [3(*R*), 5(*S*), S(*R*)]-3,5-dihydroxy-6-paratolylsulfinyl-hexanoate de méthyle (formule IV),
- le [3(*R*), 5(*S*), S(*R*)]-3,5-dioxyisopropylidène-6-paratolylsulfinyl-hexanoate de méthyle (formule V),
- le [3(*R*), 5(*S*)]6-acétoxy-syn-3,5-dioxyisopropylidène-6-paratolylthio-hexanoate de méthyle (formule VI),
- le [3(*R*), 5(*S*)]6-oxo-syn-3,5-dioxyisopropylidène-hexanoate de méthyle (formule VII),

On donne ci-après à titre d'exemple, afin d'illustrer l'objet de l'invention, sans pour autant limiter sa portée, le procédé permettant d'accéder au (4*R*,6*R*)-(+)-4-hydroxy-6-(2-phényléthyl)-tétrahydro-2H-pyran-2-one (**2**). Un schéma correspondant à ce procédé est donné dans les annexes 1 et 2.

### Exemples :

Les spectres de RMN ont été faits sur un appareil de fréquence 200MHz, les mesures de pouvoir rotatoire sont faites à 25°C.

### Exemple 1 : Synthèse du R-(+)-3,5-dioxo-6-p-tolylsulfinyl-hexanoate de méthyle (4):

A une suspension de 2,2g de NaH (0,95mole) dans 250ml de tétrahydrofuranne, maintenue à 0°C, est additionné au goutte à goutte 12,1g (0,0765mole) de 3,5-dioxo-hexanoate de méthyle (6) en solution dans 25ml de tétrahydrofurane. Le mélange devient une suspension blanche épaisse à laquelle on additionne en 15min, à 0°C, à l'aide d'une cannule, 10g d'une solution 1,5M de t-butyl lithium dans du pentane (0,15mole de t-butyl lithium). Au cours de l'addition, la solution devient jaune, puis orange, puis enfin rouge. 11,31g de (-) menthyl (S)-para-toluènesulfinate (0,038mole) en solution dans 35ml de tétrahydrofurane est ajouté goutte à goutte pendant 20min. On agite encore le mélange pendant environ 40min à 0°C, jusqu'à disparition du sulfinate. Le milieu est alors dilué par addition de 10ml d'une solution aqueuse saturée de NH₄Cl, puis de 250ml d'acétate d'éthyle et acidifié à pH 1 par addition de 170ml d'une solution aqueuse d'acide chlorhydrique 1N puis d'acide sulfurique concentré. La phase aqueuse est extraite trois fois par 150ml d'acétate d'éthyle. Les phases organiques sont rassemblées pour donner une solution qui est lavée avec 100ml de saumure, séchée sur sulfate de magnésium, puis le milieu est concentré sous vide. Le résidu est purifié par chromatographie sur gel de silice (hexane/CH₂Cl₂) puis cristallisé (CH₂Cl₂/(CH₃CH₂)₂O) pour donner 7,73g de cristaux de couleur jaune pâle.
rendement : 68%
pouvoir rotatoire : [α]_{D}=+261 (c=0,98 ;CHCl₃)
point de fusion : 55-56°C
spectre RMN H¹ (CDCl₃) : ce produit est entièrement sous forme énol
δ=14,51(s large, 1H) ; δ=7,51(fragment A d'un système (AB)₂, 2H, J_{AB}=8Hz, Δν=37Hz) ; δ=7,32(fragment B d'un système (AB)₂, 2H, J_{AB}=8Hz, Δν=37Hz) ; δ=5,65(s, 1H) ; δ=3,72(s, 3H) ; δ=3,69(fragment A d'un système AB, 1H, J_{AB}=14Hz, Δν=17Hz) ; δ=3,58(fragment B d'un système AB, 1H, J_{AB}=14Hz, Δν=17Hz) ; δ=3,35(s, 2H) ; δ=2,40(s, 3H).
spectre RMN C¹³ (CDCl₃) :
δ=188,9 ; δ=179,6 ; δ=167,4 ; δ=142,4 ; δ=139,6 ; δ=130,2 ; δ=124,1 ; δ=102,8 ; δ=64,9 ; δ=52,6 ; δ=45,3 ; δ=21,6.

| analyse élémentaire (C₁₄H₁₆O₅S) : | | |
|---|---|---|
| | C | H |
| théorique | 56,7 | 5,4 |
| trouvé | 56,7 | 5,5 |

### Exemple 2 : Synthèse du [5(S), S(R)]-(+)-5-hydroxy-3-oxo-6-paratolylsulfinyl-hexanoate de méthyle (7) :

A une solution de 5,88g (0,0198mole) du produit 4 dans 350ml de tétrahydrofuranne est additionné, au goutte à goutte, à -78°C, 40,5mL d'une solution 1M d'hydrure de diisobutyl aluminium (DIBAL-H). Après 15min d'agitation sont additionnés 80ml de méthanol. Au bout d'une heure on laisse le mélange revenir à température ambiante puis les solvants sont évaporés sous vide pour donner une fine poudre orange que l'on dissout dans 300ml d'acétate d'éthyle. A cette solution est ajouté 20ml d'une solution aqueuse saturée de disodium L-tartrate dihydrate. Ce mélange est agité pendant 12 heures à température ambiante puis acidifié à pH 4 par addition d'acide chlorhydrique concentré. La phase aqueuse est extraite deux fois par 150ml d'acétate d'éthyle puis saturée de NaCI, puis extraite une fois par 150ml d'acétate d'éthyle. Les phases organiques sont assemblées, puis cette solution est lavée avec de la saumure, séchée sur sulfate de magnésium, et le milieu est concentré sous vide. Le résidu est purifié par chromatographie sur gel de silice (hexane/CH₂Cl₂ : 1/1) puis cristallisé (CH₂Cl₂/(CH₃CH₂)₂O).pour donner 2,61g de cristaux, avec un excès énantiomérique e.e.>98%.
rendement : 44%
pouvoir rotatoire : [α]_{D}=+213 (c=1,48 ;CHCl₃) ; [α]_{D}=+153 (c=0,90 ;acétone)
point de fusion : 120-121°C
spectre RMN H¹ (CDCl₃) : 9% de forme énol identifiée par un doublet à 4,12ppm, J=3,4Hz.
δ=7,52(fragment A d'un système (AB)₂, 2H, J_{AB}=8Hz, Δν=33Hz) ; δ=7,35(fragment B d'un système (AB)₂, 2H, J_{AB}=8Hz, Δν=33Hz) ; δ=4,62(m, 1H, partie X d'un système ABX) ; δ=4,21(d, 1H, J=2Hz) ; δ=3,71(s, 3H) ; δ=2,90(partie AB d'un système ABX, 2H,J_{AB}=13,4Hz, J_{AX}=9.4Hz, J_{BX}=2,4Hz, Δν=59Hz) ; δ=3,47(s, 2H) ; δ =2,79(d, 2H, J=6Hz) ; δ=2,42(s 3H).
spectre RMN C¹³ (CDCl₃) :
δ=201,5 ; δ=167,2 ; δ=141,8 ; δ=139,2 ; δ=130,2 ; δ=124,0 ; δ=63,5 ; δ=60,4; δ=52,5 ; δ=49,6 ; δ=49,0; δ=21,4.

| analyse élémentaire (C₁₄H₁₈O₅S) : | | |
|---|---|---|
| | C | H |
| théorique | 56,4 | 6,1 |
| trouvé | 56,3 | 6,2 |

### Exemple 3 : Synthèse du [3(R), 5(S), S(R)]-(+)-3,5-dihydroxy-6-paratolylsulfinyl-hexanoate de méthyle (8) :

A une solution de 1,089g (3,65mmole) du produit 7 dans un mélange de 35ml de tétrahydrofurane et de 7ml de méthanol refroidie à -78°C est additionné rapidement 4ml d'une solution 1M de diéthylméthoxyborane (4mmole) dans du tétrahydrofurane. Cette addition provoque l'apparition d'un précipité blanc. Le mélange est agité pendant 20min puis on additionne 178mg de NaBH₄ (4,75mmole) en une seule fois, ce qui conduit à l'obtention d'une solution homogène. Après 4h d'agitation à -78°C, on laisse la solution revenir à température ambiante et on additionne 4ml d'acide acétique et 60ml d'une solution aqueuse saturée de NaHCO3, ce qui donne une solution de pH7. La phase aqueuse est extraite trois fois par 150ml d'acétate d'éthyle. Les phases organiques sont assemblées, puis cette solution est lavée avec de la saumure, séchée sur sulfate de magnésium, et le milieu est concentré sous vide. Le résidu est une huile jaune que l'on distille trois fois par distillation azéotropique avec du méthanol et l'huile obtenue est purifiée par chromatographie sur gel de silice (acétate d'éthyle) puis on la fait cristalliser à faible température (4°C) pour obtenir 1,09g de cristaux, avec un excès diastéréoisomérique d.e.>98%.
rendement : 99,4%
pouvoir rotatoire : [α]_{D}=+220 (c=1,03 ;CHCl₃) ; [α]_{D}=+219 (c=0,49 ; CHCl₃)
point de fusion : 92-94°C
spectre RMN H¹ (CDCl₃) :
δ=7,47(fragment A d'un système (AB)₂, 2H, J_{AB}=8Hz, Δν=39,6Hz) ; δ=7,27(fragment B d'un système (AB)₂, 2H, J_{AB}=8Hz, Δν=39,6Hz) ; δ=5,05(s large, 1H) ; δ=4,43(m, 1H) ; δ=4,27(m, 2H) ; δ=3,61(s, 3H) ; δ=2,83(partie AB d'un système ABX, 2H,J_{AB}=13,2Hz, J_{AX}=9.8Hz, J_{BX}=2,5Hz, Δν=30Hz) ; δ =2,43(d, 2H, J=6Hz) ; δ=2,35(s, 3H) ; δ=1,76-1,49(m, 2H).
spectre RMN C¹³ (CDCl₃) :
δ=172,9 ; δ=142,2 ; δ=140,3 ; δ=130,6 ; δ=124,5 ; δ=68,1 ; δ=66,5; δ=64,2 ; δ=52,3 ; δ=42,7 ; δ=42,2; δ=21,9.

| analyse élémentaire (C₁₄H₂₀O₅S) : | | |
|---|---|---|
| | C | H |
| théorique | 56,0 | 6,7 |
| trouvé | 56,2 | 6,7 |

### Exemple 4 : Synthèse du [3(R), 5(S), S(R)]-(+)-syn-3,5-dioxyisopropylidène-6-paratolylsulfinyl-hexanoate (9) de méthyle :

On dissout, dans 62ml d'acétone et 6,25ml de 2,2-diméthoxypropane, 1,17g du dihydroxysulfoxyde **8** (3,89mmole) et 15mg d'acide para-toluènesulfonique (0,13 % en poids). On agite le mélange à température ambiante pendant 3h jusqu'à disparition du produit de départ. Les solvants sont évaporés sous vide et le produit brut est dilué avec 100ml de dichlorométhane. A cette solution on ajoute 10ml d'une solution aqueuse saturée de NaHCO3. Le mélange est agité pendant 15min à température ambiante puis dilué avec 50ml d'eau. La phase aqueuse est extraite deux fois par 50ml de dichlorométhane et l'ensemble des phases organiques est assemblé, lavé avec 100ml d'eau, séché sur sulfate de magnésium et concentré pour donner 1,3g du produit 9 sous forme de cristaux.
rendement : 98%
pouvoir rotatoire : [α]_{D}=+195 (c=1,37 ;CHCl₃)
point de fusion : 110-111°C
spectre RMN H¹ (CDCl₃) :
δ=7,43(fragment A d'un système (AB)₂, 2H, J_{AB}=8Hz, Δν=42,2Hz) ; δ=7,21(fragment B d'un système (AB)₂, 2H, J_{AB}=8Hz, Δν=42,2Hz) ; δ=4,47-4,31(m, 1H) ; δ=4,30-4,14(m, 1H) ; δ=3,53(s, 3H) ; δ=2,43(m, 2H) ; δ=2,32(partie AB d'un système ABX, 2H,J_{AB}=15,6Hz, J_{AX}=7 Hz, J_{BX}=6Hz, Δν=37,5Hz) ; δ=2,26(s, 3H) ; δ=1,49(dt, 1H, J_{gem}=12Hz, ³J=2Hz), δ=1,4(s, 3H) ; δ=1,27(s, 3H) ; δ=1,27-1,05(m, 1H).
spectre RMN C¹³ (CDCl₃) :
δ=171,2 ; δ=141,9 ; δ=141,8 ; δ=130,3 ; δ=124,1 ; δ=99,8 ; δ=66,0; δ=63,7 ; δ=65,0 ; δ=51,9 ; δ=41,3 ; δ=36,2 ; δ=30,2 ; δ=21,7 ; δ=20,0 ;.

| analyse élémentaire (C₁₇H₂₄O₅S) : | | |
|---|---|---|
| | C | H |
| théorique | 60,0 | 7,1 |
| trouvé | 60,0 | 7,3 |

### Exemple 5 : Synthèse du [3(R), 5(S)]6-acétoxy-syn-3,5-dioxyisopropylidène-6-paratolylthio-hexanoate de méthyle (10):

On additionne 4,4g d'acétate de sodium anhydre (52,8mmole) à 1,8g (5,28mmole) du sulfoxyde **9**. 130ml d'anhydride acétique est ajouté au mélange et l'ensemble est porté au reflux pendant 10h à 135°C. Après refroidissement le milieu est filtré sur celite et le solvant est évaporé par distillation azéotropique avec du toluène. Le solide brun que l'on récupère est dilué dans 50ml de dichlorométhane et filtré sur celite. Le produit brut est purifié par chromatogrphie sur gel de silice (CH₂Cl₂) pour donner une huile brune, mélange des deux isomères en position C₆ en proportion 54/46.
spectre RMN H¹ (CDCl₃) :
   δ=7,35(fragment A d'un système (AB)₂, 2H, J_{AB}=8Hz, Δν=56,2Hz) ; δ=7,07(fragment B d'un système (AB)₂, 2H, J_{AB}=8Hz, Δν=56,2Hz) ; δ=6(d, 1H, J=5,4Hz, isomère majoritaire) ; δ=5,94(d, 1H, J=5,4Hz, isomère minoritaire) ; δ=4,30-4,15(m, 1H) ; δ=4,10-3,90(m, 1H) ; δ=3,64(s, 3H); δ=2,45(partie AB d'un système ABX, 2H,J_{AB}=15,8Hz, J_{AX}=7 Hz, J_{BX}=6Hz, Δν=36,5Hz) ; δ=2,28(s, 3H) ; δ=2,01(s, 3H) ; δ=1,74(dt, 1H, J_{gem}=12,8Hz, ³J=2,4Hz, isomère majoritaire) ; δ=1,65(dt, 1H, J_{gem}=14,4Hz, ³J=2,4Hz, isomère minoritaire), δ=1,38(s, 3H, isomère majoritaire) ; δ=1,34(s, 3H, isomère majoritaire) ; δ=1,36(s, 3H, isomère minoritaire) ; δ=1,34(s, 3H, isomère minoritaire) ; δ=1,46-1,22(m, 1H).
spectre RMN C¹³ (CDCl₃) :
   δ=171,6 ; δ=170,2 ; δ=170,0 ; δ=139,1; δ=139,0 ; δ=134,7 ; δ=134,3 ; δ=130,3 ; δ=130,2 ; δ=128,6 ; δ=128,2 ; δ=99,9 ; δ=99,8 ; δ=83,3 ; δ=83,1 ; δ=70,8 ; δ=70,1 ; δ=66,0 ; δ=65,9 ; δ=51,2 ; δ=41,5 ; δ=33,1 ; δ=32,6 ; δ=30,3 ; δ=21,7 ; δ=21,5 ; δ=21,4 ; δ=20,0.

### Exemple 6 : Synthèse du [3(R), 5(S)]-(+)-6-hydroxy-syn-3,5-dioxyisopropylidène-hexanoate de méthyle (11):

### 1)première étape :

A une solution de 1,29g (3,37mmole) de **10** dans 90ml d'éthanol est ajouté du nickel de Raney par portions, à température ambiante. La réaction est suivie par chromatographie sur plaque de silice (éluant : AcOEt/CH₂Cl₂ : 1/1). Le mélange est filtré sur celite et le solide lavé au méthanol. On récupère la phase liquide et après évaporation sous vide des solvants, le résidu est purifié sur colonne de silice (éluant : AcOEt/CH₂Cl₂ : 1/1). On récupère 0,641g de cristaux blancs de [3(R), 5(S)]-(+)-6-acétoxy-3,5-dioxyisopropylidène-hexanoate de méthyle.

### 2)deuxième étape :

706mg de ce produit sont repris dans 70ml de méthanol. On ajoute au goutte à goutte à cette solution 927mg de K₂CO₃ en solution dans 6ml d'eau. La solution initialement incolore tourne au jaune et il se forme un précipité blanc. Après avoir agité pendant 3h à température ambiante on ajoute 50ml d'eau et du NH₄Cl, jusqu'à l'obtention d'un pH7. La phase aqueuse est extraite trois fois par de l'acétate d'éthyle, puis elle est saturée par du NaCI et extraite à nouveau trois fois par de l'acétate d'éthyle. Les phases organiques sont réunies, séchées sur sulfate de magnésium, lavées, et les solvants sont évaporés sous vide pour donner 459mg du produit **11**, sous la forme d'un liquide incolore.
rendement : 78%
pouvoir rotatoire : [α]_{D}=+9,7 (c=1,85 ;CHCl₃)
spectre RMN H¹ (CDCl₃) :
δ=4,33(tdd, 1H, J=11,4Hz, J=6,4Hz, J=2,6 Hz) ; δ=4,00(m, 1H) ; δ=3,67(s, 3H) ; δ=3,53(partie AB d'un système ABX, 2H,J_{AB}=11,5Hz, J_{AX}=6Hz, J_{BX}=2,5Hz, Δν=26Hz) ; δ=2,47(partie AB d'un système ABX, 2H,J_{AB}=15,6Hz, J_{AX}=6,9Hz, J_{BX}=6,1Hz, Δν=39Hz) ; δ=2,18(s large, 1H) ; δ=1,50(dt, 1H, J_{gem}=13,2Hz, ³J=2,8Hz), δ=1,46(s, 3H) ; δ=1,37(s, 3H) ; δ=1,40-1,21(m, 1H).
spectre RMN C¹³ (CDCl₃) :
δ=171,8 ; δ=99,5 ; δ=70,1 ; δ=66,3 ; δ=66,0 ; δ=52,1 ; δ=41,6 ; δ=32,4 ; δ=30,4 ; δ=20,2.

| analyse élémentaire (C₁₀H₁₈O₅) : | | |
|---|---|---|
| | C | H |
| théorique | 55,0 | 8,3 |
| trouvé | 54,9 | 8,2 |

### Exemple 7 : Synthèse du (3(R), 5(R)]-(+)-7phényl-3,5-dioxyisopropylidène-heptanoate de méthyle (12) :

1)première étape : 0,320ml de DMSO est ajouté au goutte à goutte à une solution de 0,2ml de chlorure d'oxalyle dans 5ml de dichlorométhane à -75°C. Après 10min, 156,9mg (0,718mmole) de l'alcool 11 dilué dans 5ml de dichlorométhane est ajouté lentement à cette solution. Le mélange, hétérogène, est agité pendant 1,5h et la température augmente jusqu'à -40°C. Le mélange est refroidi à -50°C avant d'ajouter 1,2ml de triéthylamine, puis on laisse le mélange revenir à la température ambiante sous agitation pendant 1h. 4ml de dichlorométhane sont ajoutés pour solubiliser le mélange. L'avancement de la réaction est suivi par chromatographie sur plaques de silice. Lorsque la réaction est finie, le milieu est dilué avec 20ml de dichlorométhane et 20ml d'une solution aqueuse saturée de NH₄Cl, puis neutralisé avec 2ml d'une solution aqueuse à 10% d'acide chlorhydrique. La phase aqueuse est lavée trois fois à l'eau et avec de la saumure puis séchéesur sulfate de magnésium. Les solvants sont évaporés sous vide pour donner 125mg d'une huile jaune : le [3(R), 5(S)]-(+)-6-oxo-syn-3,5-dioxyisopropylidène-hexanoate de méthyle.

2)deuxième étape : A une solution contenant 204mg de bromure de benzyl triphénylphosphonium dans 5ml de tétrahydrofurane anhydre est additionnée 0,320ml d'une solution de n-butyl lithium 1,5M dans l'hexane. Le mélange est agité pendant 10min puis on ajoute une solution de 90mg de l'aldéhyde préparé à l'étape précédente dans 10ml de tétrahydrofurane anhydre. Le milieu est agité pendant 4h à température ambiante pour donner une solution jaune avec un précipité blanc. On ajoute ensuite 50ml de dichlorométhane et 60ml d'une solution aqueuse saturée de NH₄Cl de sorte que l'on ajuste le pH à 6. La phase aqueuse est extraite deux fois par 50ml de dichlorométhane et la phase organique est séchée sur sulfate de magnésium, puis les solvants sont évaporés et le produit brut est purifié par chromatographie sur colonne de silice (éluant : CH₂Cl₂). On récupère 79,1mg de [3(R), 5(S)]-(+)-7-phényl-syn-3,5-dioxyisopropylidène-6-heptenoate de méthyle sous la forme d'un mélange 85/15 d'isomère Z/E.

### 3) troisième étape :

Le produit préparé à l'étape précédente (30,4mg, 0,104mmole) est réduit en solution dans 6ml d'acétate d'éthyle sous hydrogène en présence de 25mg de palladium sur charbon (5%) pendant 24h. Le mélange est alors filtré sur celite, lavé au dichlorométhane et concentré pour donner 29,6mg de [3(R), 5(R)]-(+)-7phényl-3,5-dioxyisopropylidène-heptanoate de méthyle (**12**) sous la forme d'un liquide incolore.
rendement (trois étapes): 51%
pouvoir rotatoire : [α]_{D}=+23 (c=1,45 ;CHCl₃)
spectre RMN H¹ (CDCl₃):
δ=7,32-7,13(m, 5H) ; δ=4,26(tdd, 1H, Hx d'un système ABX, J=12Hz, J=6,5Hz, J=2,6 Hz) ; δ=3,88-3,75(m, 1H) ; δ=3,68(s, 3H) ; δ=2,82-2,61(m, 2H) ; δ=2,46(partie AB d'un système ABX, 2H, J_{AB}=15,4Hz, J_{AX}=6,7Hz, J_{BX}=6,3Hz, Δν=41Hz) ; δ=1,93-1,63(m, 2H) ; δ=1,56(dt, 1H, J_{gem}=12Hz, ³J=2,6Hz), δ=1,43(s, 3H) ; δ=1,40(s, 3H) ; δ=1,33-1,12(m, 1H).
spectre RMN C¹³ (CDCl₃) :
δ=172,1 ; δ=142,6 ; δ=129,2 ; δ=129,0 ; δ=126,4 ; δ=68,2 ; δ=66,6 ; δ=52,3 ; δ=41,9 ; δ=38,5 ; δ=37,2 ; δ=31,7 ; δ=30,8 ; δ=20,4.

| analyse élémentaire (C₁₇H₂₄O₄): | | |
|---|---|---|
| | C | H |
| théorique | 69,8 | 8,3 |
| trouvé | 69,1 | 8,5 |

### Exemple 8 : Synthèse du (4R,6R)-(+)-4-hydroxy-6-(2-phényléthyl)-tétrahydro-2H-pyran-2-one (2):

On porte une solution de 27mg de l'ester **12** dans 2ml d'acide acétique et 0,5ml d'eau à 92°C pendant 2h. Après deux heures à température ambiante, la solution est diluée par 20ml de CH2Cl2 et 10ml d'eau, puis neutralisée par une solution aqueuse saturée de NaHCO3. La phase aqueuse est extraite quatre fois par 10ml de dichlorométhane, la phase organique est lavée avec de la saumure et séchée sur sulfate de magnésium. Le produit brut est purifié par chromatographie sur silice. On récupère 16mg de **2** sous forme de cristaux que l'on fait recristalliser dans un mélange de dichlorométhane et d'hexane.
rendement : 81%
pouvoir rotatoire : [α]_{D}=+71 (c=0,94 ; CHCl₃)
point de fusion : 108°C
spectre RMN H¹ (CDCl₃) :
δ=7,09-7,44(m, 5H) ; δ=4,71(m, 1H) ; δ=4,37(m, 1H) ; δ=2,96-2,64(m, 4H) ; δ=2,17-1,69(m, 4H) ; δ=2,46(m, 1H)
spectre RMN C¹³ (CDCl₃) :
δ=171,3 ; δ=141,7 ; δ=129,2 ; δ=129,1 ; δ=126,8 ; δ=75,7 ; δ=63,3; δ=41,7 ; δ=39,3 ; δ=38,0 ; δ=31,8

| analyse élémentaire (C₁₃H₁₆O₃): | | |
|---|---|---|
| | C | H |
| théorique | 70,9 | 7,4 |
| trouvé | 70,8 | 7,1 |

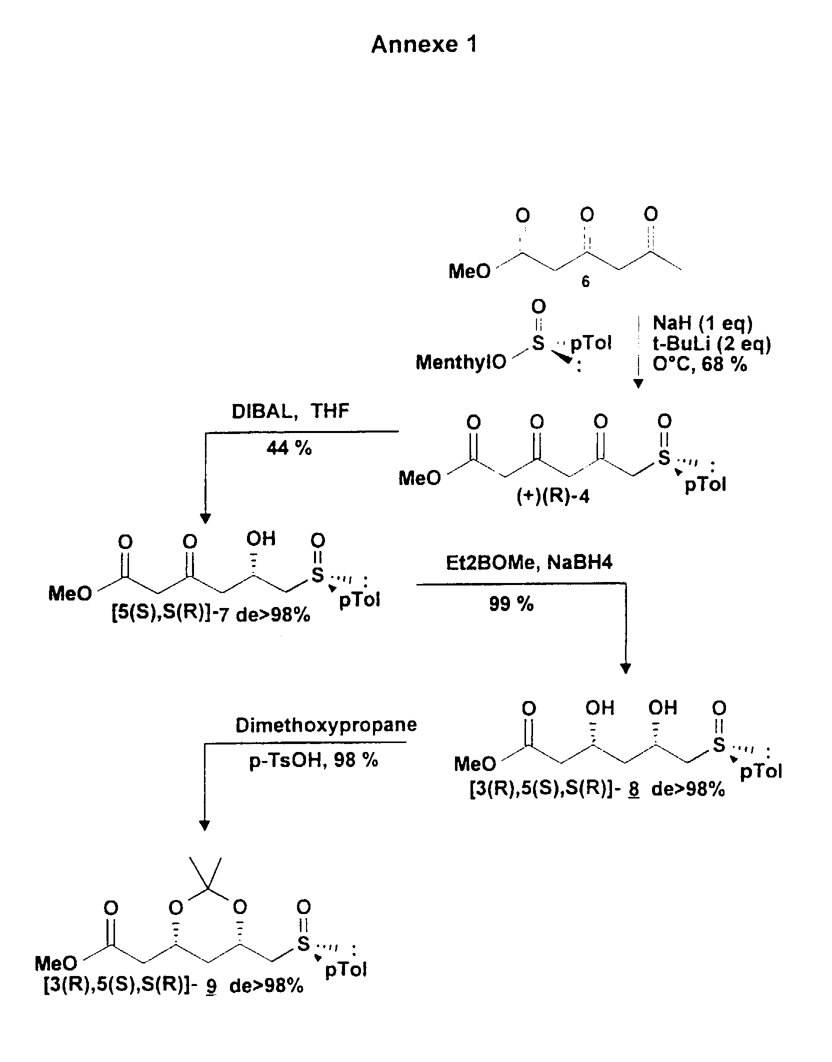

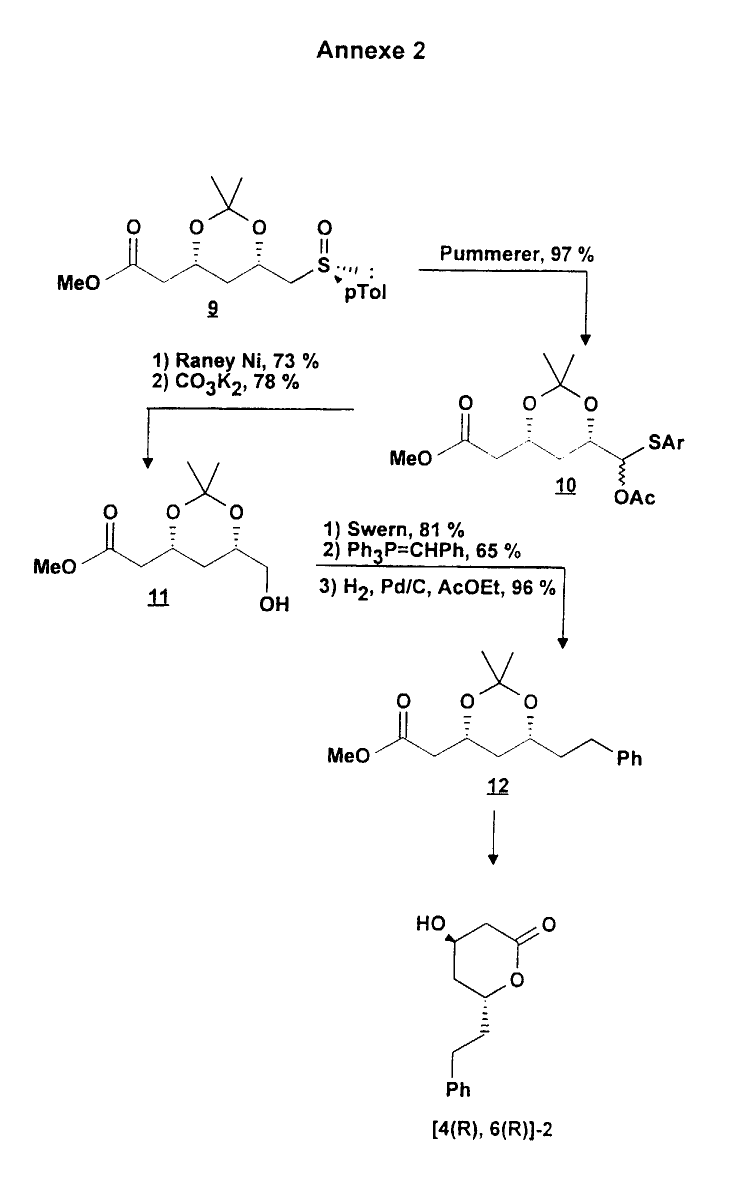

## Revendications

1. (Xs)-3,5-dioxo-6-p-tolylsulfinyl hexanoate d'alkyle répondant à la formule (II), 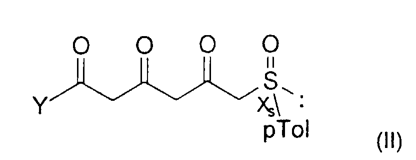 dans laquelle Xs désigne la configuration *R* ou *S* du soufre, caractérisé en ce que Y=OCH₃.

2. Produit selon la revendication 1, caractérisé en ce qu'il est le (R)-3,5-dioxo-6-p-tolylsulfinyl hexanoate de méthyle.

3. Procédé de préparation des (Xs)-3,5-dioxo-6-p-tolylsulfinyl hexanoates d'alkyle répondant à la formule (II), 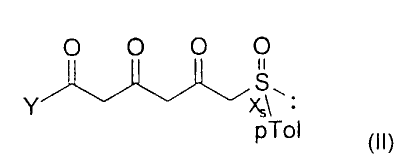 dans laquelle Xs désigne la configuration *R* ou *S* du soufre, Y représente un radical alkyloxy en C1-C18 linéaire ou ramifié, saturé ou insaturé, un radical aryloxy ou un radical aralkyloxy en C1-C18, un radical imidazolide, un radical alkylsulfure en C1-C18 linéaire ou ramifié, saturé ou insaturé, arylsulfure ou aralkylsulfure en C1-C18, un radical 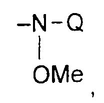 Q désignant un radical alkyle en C1-C18 linéaire ou ramifié, saturé ou insaturé, caractérisé en ce que l'on fait réagir le tri-anion du 3,5-di-oxo-hexanoate d'alkyle de formule (X):
Y-CO-CH₂-CO-CH₂-CO-CH₃ (X)
avec le (X's)-p-toluène sulfinate de (-) ou de (+) menthyle, X's désignant les configurations *R* ou *S* du soufre et X's ≠ Xs.

4. Procédé selon la revendication 3, caractérisé en ce que le tri-anion du 3,5-di-oxo-hexanoate d'alkyle est obtenu par traitement avec de l'hydrure de sodium et soit du ter-butyl lithium soit du sec-butyl lithium en milieu anhydre à basse température.

5. Procédé selon la revendication 4, caractérisé en ce que que le tri-anion du 3,5-di-oxo-hexanoate d'alkyle est obtenu par traitement avec environ un équivalent d'hydrure de sodium et deux équivalents de ter-butyl lithium ou en présence d'environ un équivalent d'hydrure de sodium et deux équivalents de sec-butyl lithium.

6. Procédé selon l'une quelconque des revendications 3 à 5, caractérisé en ce que la réaction est faite dans un solvant choisi parmi les éthers.

7. Procédé selon la revendication 5, caractérisé en ce que la réaction est faite dans le tétrahydrofurane.

8. Procédé selon l'une quelconque des revendications 3 à 7, caractérisé en ce que la réaction est effectuée à une température allant de -10°C à +10°C.

9. Procédé selon la revendication 8, caractérisé en ce que la réaction est faite à une température voisine de 0°C.

10. Procédé selon l'une quelconque des revendications 3 à 9, caractérisé en ce que cette réaction se fait avec une quantité de p-toluène sulfinate de menthyle sensiblement égale à 50% en nombre de moles par rapport au nombre de moles de 3,5-di-oxo-hexanoate d'alkyle.

11. Procédé selon l'une quelconque des revendications 3 à 10, caractérisé en ce que on utilise le (*S*)-p-toluène sulfinate de (-) menthyle ou le (*R*)-p-toluène sulfinate de (+) menthyle.

12. Procédé selon la revendication 10, caractérisé en ce que on utilise le (*S*)-p-toluène sulfinate de (-) menthyle.

13. [5(S), S(R)]-5-hydroxy-3-oxo-6-paratolylsulfinyl-hexanoate de méthyle.

14. Procédé de préparation des [5(X₅), S(Xs)]-5-hydroxy-3-oxo-6-paratolylsulfinyl-hexanoates d'alkyle répondant à la formule (III), 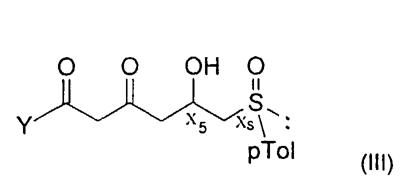 X₅ désignant la configuration *R* ou *S* du carbone C₅, avec X₅ ≠ Xs, Y ayant la même signification que dans la revendication 3, Xs désignant la configuration *R* ou *S* du soufre, caractérisé en ce que l'on réduit en alcool de façon énantiosélective la fonction 5-carbonyle du (Xs)-3,5-di-oxo-6-paratolylsulfinyl hexanoate d'alkyle répondant à la formule (II): 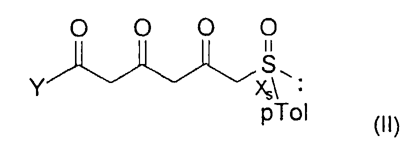 dans laquelle Xs désigne la configuration *R* ou *S* du soufre.

15. Procédé selon la revendication 14, caractérisé en ce que la réduction est faite par un hydrure respectant l'induction stéréochimique due au radical (Xs)-paratolylsulfinyle et ne réduisant pas le groupement -COY.

16. Procédé selon l'une quelconque des revendications 14 et 15, caractérisé en ce que l'on utilise l'hydrure de di-isobutyl aluminium.

17. Procédé selon l'une quelconque des revendications 14 à 16, caractérisé en ce que la réaction est conduite en milieu anhydre, les réactifs étant introduits à une température inférieure à -20°C et on laisse ensuite le milieu revenir à température ambiante.

18. Procédé selon l'une quelconque des revendications 14 à 17, caractérisée en ce que la réaction est faite dans le tétrahydrofurane et les réactifs sont introduits à une température de -50°C.

19. Procédé selon l'une quelconque des revendications 14 à 18, caractérisé en ce que les réactifs sont introduits à une température aux environs de -78°C.

20. [3 (X₃), 5(X₅), S(Xs)]-3,5-dihydroxy-6-paratolylsulfinyl-hexanoate d'alkyle de formule (IV), 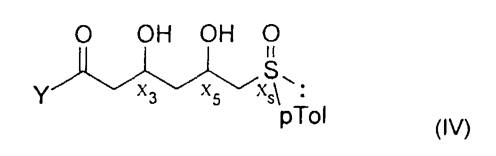 X₃ désignant la configuration *R* ou *S* du carbone C₃, Y ayant la même signification que dans la revendication 3, Xs désignant la configuration *R* ou *S* du soufre, X₅ désignant la configuration *R* ou *S* du carbone C₅, avec X₅ ≠ Xs.

21. [3 (X₃), 5(X₅), S(Xs)]-3,5-dihydroxy-6-paratolylsulfinyl-hexanoate d'alkyle selon la revendication 20, caractérisés en ce que X₅ = X₃.

22. Procédé de préparation des produits selon les revendications 20 et 21, caractérisé en ce que l'on réduit la fonction 3-carbonyle d'un [5(X₅), S(Xs)]-5-hydroxy-3-oxo-6-paratolylsulfinyl-hexanoates d'alkyle répondant à la formule (III), 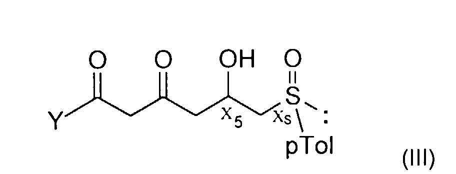 X₅ désignant la configuration *R* ou *S* du carbone C₅, avec X₅ ≠ Xs, Y ayant la même signification que dans la revendication 3, Xs désignant la configuration *R* ou *S* du soufre, par un traitement avec du triacétoxyborohydrure de tétraméthylammonium.

23. [3 (X₃), 5(X₅), S(Xs)]-3,5-dihydroxy-6-paratolylsulfinyl-hexanoate d'alkyle selon la revendication 20, caractérisés en ce que X₅ ≠ X₃.

24. Produit selon la revendication 23, caractérisé en ce qu'il est le [3(*R*), 5(*S*), S(*R*)]-3,5-dihydroxy-6-paratolylsulfinyl-hexanoate de méthyle,

25. Procédé de préparation des produits selon les revendications 23 et 24, caractérisé en ce que l'on traite un [5(X₅), S(Xs)]-5-hydroxy-3-oxo-6-paratolylsulfinyl-hexanoate d'alkyle répondant à la formule (III), 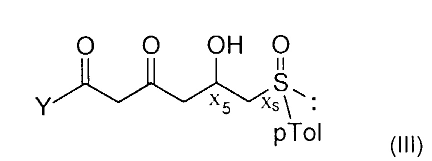 X₅ désignant la configuration *R* ou *S* du carbone C₅, avec X₅ ≠ Xs, Y ayant la même signification que dans la revendication 3, Xs désignant la configuration *R* ou *S* du soufre, par du borohydrure de sodium en présence d'un agent chélatant.

26. Procédé selon la revendication 25, caractérisé en ce que l'agent chélatant est un dérivé du zinc du titane ou du bore.

27. Procédé selon la revendication 26, caractérisé en ce que l'agent chélatant est choisi parmi le tétraisopropyloxytitane, le bromure de zinc et le diéthylméthoxyborane.

28. Procédé selon la revendication 27, caractérisé en ce que l'agent chélatant est du diéthylméthoxyborane.

29. Procédé selon l'une quelconque des revendications 25 à 28, caractérisé en ce que la réaction est conduite en milieu anhydre.

30. Procédé selon l'une quelconque des revendications 25 à 29, caractérisé en ce que les réactifs sont introduits à une température inférieure à 0°C.

31. Procédé selon l'une quelconque des revendications 25 à 30, caractérisé en ce que la réaction est mise en oeuvre en présence d'un excès de diéthylméthoxyborane et de borohydrure de sodium.

32. Procédé selon l'une quelconque des revendications 25 à 31, caractérisé en ce que la réaction est faite dans le tétrahydrofurane en introduisant les réactifs à une température inférieure à -20°C et préférentiellement à -78°C.

33. [3(X₃), 5(X₅), S(Xs)]-3,5-di-alkyloxy-6-paratolylsulfinyl-hexanoate d'alkyle de formule (V), 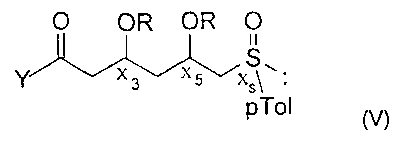 dans laquelle les deux fonctions hydroxyles sont protégées par des radicaux alkyle en C1-C8 ou aryle ou aralkyle, des radicaux tri-alkylsilyle, ou par un radical alcane di-yle en C1-C8, un radical benzylidène, radicaux désignés R , Y et ayant la même signification que dans la revendication 3, Xs désignant la configuration *R* ou *S* du soufre, X₃ ayant la même signification que dans la revendication 20, X₅ désignant la configuration *R* ou *S* du carbone C₅, avec X₅ ≠ Xs.

34. [3(X₃), 5(X₅), S(Xs)]-3,5-di-alkyloxy-6-paratolylsulfinyl-hexanoate d'alkyle selon la revendication 33, caractérisé en ce que R est choisi parmi les radicaux méthyle, benzyle, terbutyldiméthylsilyle, terbutyldiphénylsilyle, isopropylidène et benzylidène, ces deux derniers radicaux permettant à eux seuls de protéger simultanément les deux hydroxyles.

35. [3(X₃), 5(X₅), S(Xs)]-3,5-di-alkyloxy-6-paratolylsulfinyl-hexanoate d'alkyle selon la revendication 34, caractérisé en ce que R est le radical isopropylidène.

36. Produit selon la revendication 35, caractérisé en ce qu'il est le [3(*R*), 5(*S*), S(*R*)]-3,5-dioxyisopropylidène-6-paratolylsulfinyl-hexanoate de méthyle,

37. Procédé de préparation du produit selon les revendications 35 et 36, caractérisé en ce que l'on traite le [3(X₃), 5(X₅), S(Xs)]-3,5-di-hydroxy-6-paratolylsulfinyl-hexanoate d'alkyle par du diméthoxypropane en présence de quantités catalytiques d'acide para toluène sulfonique dans l'acétone.

38. [3(X₃), 5(X₅)]-6-acétoxy-3,5-di-alkyloxy-6-paratolyl-thio-hexanoate d'alkyle de formule (VI) 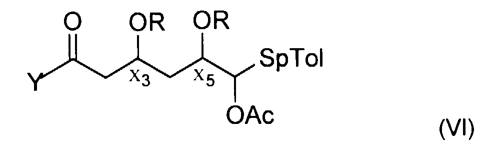 dans laquelle Y a la même signification que dans les revendications 1 à 3, R a la même signification que dans la revendication 33, X₃ a la même signification que dans la revendication 20 et X₅ désigne la configuration *R* ou *S* du carbone C₅.

39. Produit selon la revendication 38, caractérisé en ce qu'il est le [3(*R*), 5(*S*)]6-acétoxy-syn-3,5-dioxyisopropylidène-6-paratolylthio-hexanoate de méthyle.

40. Procédé de préparation de [3(X₃), 5(X₅)] 6-hydroxy 3,5-di-alkyloxy-hexanoate d'alkyle répondant à la formule (XI) : 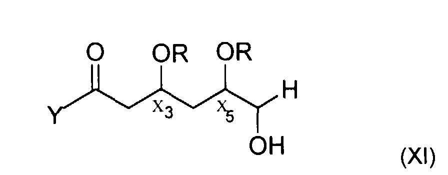 dans laquelle Y a la même signification que dans la revendication 3, R a la même signification que dans la revendication 33, X₃ a la même signification que dans la revendication 20 et X₅ désigne la configuration *R* ou *S* du carbone C₅, caractérisé en ce que l'on réduit la fonction thioéther et que l'on hydrolyse l'acétate d'un produit selon l'une quelconque des revendications 38 et 39.

41. Procédé selon la revendication 40, caractérisé en ce que l'on fait un traitement au nickel de Raney dans un solvant protique ou dans l'acétate d'éthyle et que l'acétate est éliminé en traitant le milieu par une base.

42. Procédé selon la revendication 41, caractérisé en ce que l'acétate est éliminé par un traitement au carbonate ou à l'hydrure de di-isobutyl aluminium.

43. Procédé de préparation de [3(X₃), 5(X₅)] 6-oxo 3,5-di-alkyloxy-hexanoate d'alkyle répondant à la formule (VII) : 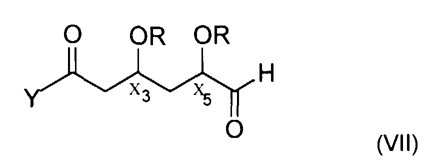 dans laquelle Y a la même signification que dans la revendication 3, R a la même signification que dans la revendication 33, X₃ a la même signification que dans la revendication 20 et X₅ désigne la configuration *R* ou *S* du carbone C₅, avec X₅ ≠ Xs, caractérisé en ce que l' on traite le produit selon la revendication 38 ou 39 par du carbonate en milieu aqueux.

44. Procédé de préparation de produits répondant à l'une des formules (la) et (Ib) 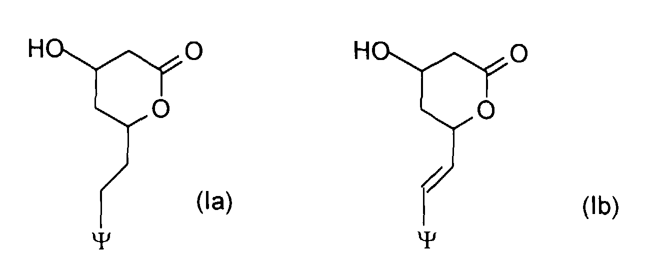 dans laquelle Ψ a la même signification que ci-dessous, caractérisé en ce qu'il comprend au moins une étape choisie parmi les étapes 1 à 8 suivantes :
1) dans la première étape, on fait réagir le tri-anion du 3,5-di-oxo-hexanoate d'alkyle de formule (X) avec le (X's)-p-toluène (noté pTol) sulfinate de (-) ou de (+) menthyle pour obtenir le (Xs)-3,5-di-oxo-6-p-tolylsulfinyl hexanoate d'alkyle de formule (II),
Y-CO-CH₂-CO-CH₂-CO-CH₃ (X) 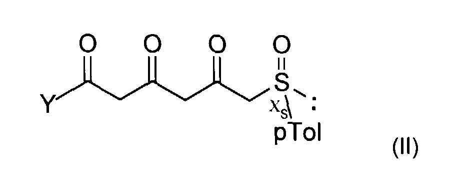
Xs et X's désignant les configurations *R* ou *S* du soufre dans les deux molécules, avec Xs ≠ X's, Y représentant un radical alkyloxy en C1-C18 linéaire ou ramifié, saturé ou insaturé, un radical aryloxy ou un radical aralkyloxy en comprenant au plus 18 atomes de carbone , un radical imidazolide, un radical alkylsulfure en C1-C18 linéaire ou ramifié, saturé ou insaturé, arylsulfure, aralkylsulfure en C1-C20, un radical 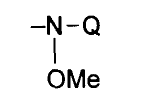 Q désignant un radical alkyle en C1-C18 linéaire ou ramifié, saturé ou insaturé
2) dans la seconde étape, on réduit de façon énantiosélective la fonction 5-carbonyle du (Xs)-3,5-di-oxo-6-paratolylsulfinyl hexanoate d'alkyle en alcool pour donner le [5(X₅), S(Xs)]-5-hydroxy-3-oxo-6-paratolylsulfinyl-hexanoate d'alkyle de formule (III) ci-après, X₅ désignant la configuration du carbone C₅, avec X₅ ≠ Xs et Y ayant la même signification que ci-dessus, 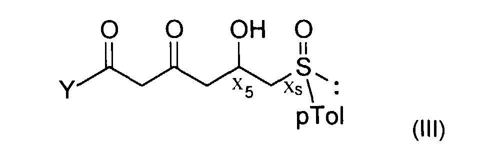
3) dans la troisième étape, on réduit de façon diastéréosélective la fonction 3-carbonyle du [5(X₅), S(Xs)]-5-hydroxy-3-oxo-6-paratolylsulfinyl-hexanoate d'alkyle pour obtenir le [3 (X₃), 5(X₅), S(Xs)]-3,5-dihydroxy-6-paratolylsulfinyl-hexanoate d'alkyle de formule (IV) ci-après, X₃ désignant la configuration du carbone C₃, en choisissant une méthode de réduction donnant X₅ = X₃ si l'on veut une configuration anti ou une méthode de réduction donnant X₅ ≠ X₃ si l'on veut une configuration syn des deux fonctions alcool, Y ayant la même signification que ci-dessus, 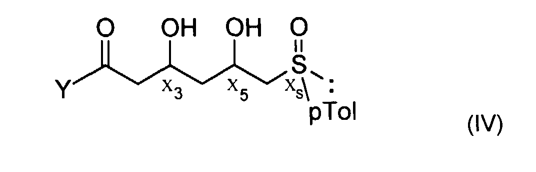
4) dans la quatrième étape, on protège les deux fonctions hydroxyle par des radicaux alkyle en C1-C8 ou aryle ou aralkyle, des radicaux tri-alkylsilyle, ou par un radical alcane di-yle en C1-C8, un radical benzylidène, radicaux que l'on désignera R, pour obtenir un [3(X₃), 5(X₅), S(Xs)]-3,5-di-alkyloxy-6-paratolylsulfinyl-hexanoate d'alkyle de formule (V) ci dessous, Y ayant la même signification que ci-dessus, lesdits radicaux n'étant pas déplacés dans les conditions des étapes 5 et 6, 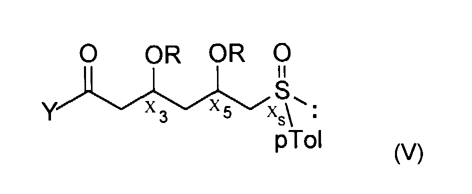
5) dans la cinquième étape, on réduit la fonction sulfoxyde en thioéther par une réaction de Pummerer pour obtenir un [3(X₃), 5(X₅)]-6-acétoxy-3,5-di-alkyloxy-6-paratolyl-thio-hexanoate d'alkyle de formule (VI) ci-après, R et Y ayant la même signification que ci-dessus, 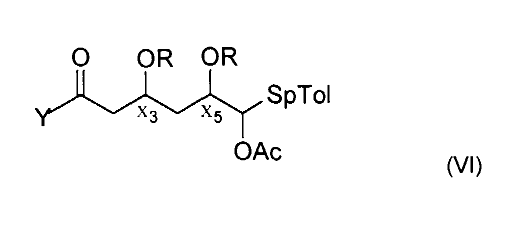
6) dans la sixième étape, on transforme les fonctions en C6 en aldéhyde pour obtenir le [3(X₃), 5(X₅)] 6-oxo 3,5-di-alkyloxy-hexanoate d'alkyle répondant à la formule (VII), Y et R ayant la même signification que ci-dessus, 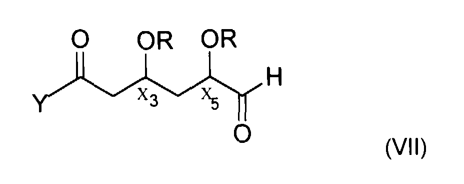
7) dans la septième étape on condense le [3(X₃), 5(X₅)] 6-oxo 3,5-di-alkyloxy-hexanoate d'alkyle de formule (VII) sur un réactif de Wittig Ph₃P=CHΨ, où Ψ représente un radical choisi parmi :
- les alkyles en C1-C40, linéaires ramifiés ou cycliques, saturés ou insaturés, éventuellement interrompus par des ponts éthers et /ou substitués par au moins un substituant choisi parmi les groupements halogène, hydroxyle, alcoxy en C1-C20, acyloxy en C1-C20, amino primaire secondaire ou tertiaire, nitro, thiol, carboxyle, amido, carboxylate d'alkyle en C1-C20, carboxylate d'aryle, carboxylate d'aralkyle en C1-C20, alkylamido en C1-C20, arylamido, aralkylamido en C1-C20 , et parmi les radicaux aryles, les hétérocycles azotés, oxygénés phosphorés et soufrésen C1-C20 , saturés ou insaturés.
- les aryles, éventuellement substitués par au moins un substituant choisi parmi les groupements halogène, hydroxyle, alcoxy en C1-C20, acyloxy en C1-C20, amino alkylamino et dialkylamino en C1-C20, nitro, thiol, carboxyle, amido, carboxylate d'alkyle en C1-C20, carboxylate d'aryle, carboxylate d'aralkyle en C1-C20 , alkylamido en C1-C20, arylamido, aralkylamido en C1-C20 , aryles en C1-C20 et parmi les radicaux alkyles et aralkyles en C1-C20 , saturés ou insaturés, linéaires, ramifiés ou cycliques, les hétérocycles azotés, oxygénés phosphorés et soufrés en C1-C20 , saturés ou insaturés.
- les aralkyles, éventuellement substitués par au moins un substituant choisi parmi les groupements halogène, hydroxyle, alcoxy en C1-C20, acyloxy en C1-C20, amino alkylamino et dialkylamino en C1-C20, nitro, thiol, acide carboxylique, amido, carboxylate d'alkyle en C1-C20, carboxylate d'aryle, carboxylate d'aralkyleen C1-C20, alkylamido en C1-C20, arylamido, aralkylamidoen C1-C20, aryles en C1-C20 et parmi les radicaux alkyles et aralkyles en C1-C20, saturés ou insaturés, linéaires, ramifiés ou cycliques.
- les hétérocycles azotés, oxygénés ou soufrés, éventuellement substitués par au moins un substituant choisi parmi les groupements halogène, hydroxyle, alcoxy en C1-C20, acyloxy en C1-C20, amino primaire secondaire ou tertiaire, nitro, thiol, acide carboxylique, amido, carboxylate d'alkyle en C1-C20, carboxylate d'aryle, carboxylate d'aralkyle en C1-C20 , alkylamido en C1-C20, arylamido, aralkylamido en C1-C20 , et parmi les radicaux aryles, aralkyles, les hétérocycles azotés, oxygénés phosphorés et soufrésen C1-C20, saturés ou insaturés,
pour donner le produit de formule (VIII), R et Y ayant la même signification que ci-dessus : 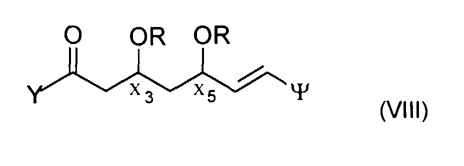
8) dans la huitième étape on réduit la double liaison pour obtenir le produit de formule (IX) suivant : 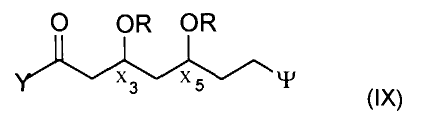
9) dans la neuvième et dernière étape on élimine les groupes protecteurs R des hydroxyle et on hydrolyse l'ester -COY du produit de formule (VIII) ou du produit de formule (IX) pour donner respectivement le produit de formule (Ib) ou le produit de formule (la).

## Claims

1. Alkyl (Xs)-3,5-dioxo-6-(p-tolylsulphinyl)-hexanoate corresponding to the formula (II), 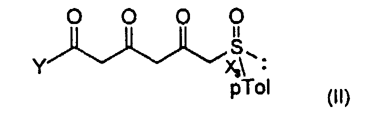 in which Xs denotes the *R* or *S* configuration of the sulphur, characterized in that Y=OCH₃.

2. Product according to Claim 1, characterized in that it is methyl (R)-3,5-dioxo-6-(p-tolylsulphinyl)hexanoate.

3. Process for preparing the alkyl (Xs)-3,5-dioxo-6-(p-tolylsulphinyl)hexanoates corresponding to the formula (II), 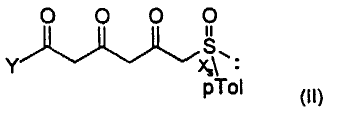 in which Xs denotes the *R* or *S* configuration of the sulphur, Y represents a saturated or unsaturated, linear or branched C₁-C₁₈ alkyloxy radical, an aryloxy radical or a C₁-C₁₈ aralkyloxy radical, an imidazolide radical, a saturated or unsaturated, linear or branched C₁-C₁₈ alkyl sulphide radical, an aryl sulphide or C₁-C₁₈ aralkyl sulphide radical or a radical 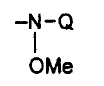 Q denoting a saturated or unsaturated, linear or branched C₁-C₁₈ alkyl radical, characterized in that the tri-anion of the alkyl 3,5-dioxohexanoate of formula (X):
Y-CO-CH₂-CO-CH₂-CO-CH₃ (X)
is reacted with (-)- or (+)-menthyl (X's)-p-toluenesulphinate, X's denoting the *R* or *S* configurations of the sulphur and X's ≠ Xs.

4. Process according to Claim 3, characterized in that the tri-anion of the alkyl 3,5-dioxohexanoate is obtained by treatment with sodium hydride and either tert-butyllithium or sec-butyllithium in an anhydrous medium at low temperature.

5. Process according to Claim 4, characterized in that the tri-anion of the alkyl 3,5-dioxohexanoate is obtained by treatment with approximately one equivalent of sodium hydride and two equivalents of tert-butyllithium or in the presence of approximately one equivalent of sodium hydride and two equivalents of sec-butyllithium.

6. Process according to any one of Claims 3 to 5, characterized in that the reaction is carried out in a solvent chosen from ethers.

7. Process according to Claim 5, characterized in that the reaction is carried out in tetrahydrofuran.

8. Process according to any one of Claims 3 to 7, characterized in that the reaction is performed at a temperature ranging from -10°C to +10°C.

9. Process according to Claim 8, characterized in that the reaction is carried out at a temperature in the region of 0°C.

10. Process according to any one of Claims 3 to 9, characterized in that this reaction is carried out with an amount of menthyl p-toluenesulphinate substantially equal to 50% in number of moles relative to the number of moles of alkyl 3,5-dioxohexanoate.

11. Process according to any one of Claims 3 to 10, characterized in that (-)-menthyl (*S*)-p-toluenesulphinate or (+)-menthyl (*R*)-p-toluenesulphinate is used.

12. Process according to Claim 10, characterized in that (-)-menthyl (*S*)-p-toluenesulphinate is used.

13. Methyl [5(S),S(R)]-5-hydroxy-3-oxo-6-(para-tolylsulphinyl)hexanoate.

14. Process for preparing the alkyl [5 (X₅),S(Xs)]-5-hydroxy-3-oxo-6-(para-tolylsulphinyl)-hexanoates corresponding to the formula (III), 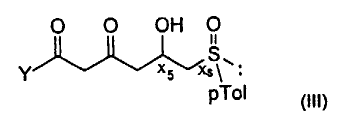 X₅ denoting the *R* or *S* configuration of the C₅ carbon, with X₅ ≠ Xs, Y having the same meaning as in Claim 3, Xs denoting the *R* or *S* configuration of the sulphur, characterized in that the 5-carbonyl function of the alkyl (Xs)-3,5-dioxo-6-(para-tolylsulphinyl)hexanoate corresponding to the formula (II): 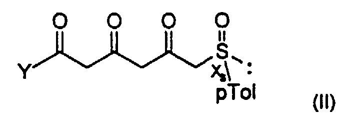 in which Xs denotes the *R* or *S* configuration of the sulphur, is reduced enantioselectively to alcohol.

15. Process according to Claim 14, characterized in that the reduction is carried out with a hydride that does not impair the stereochemical induction due to the (Xs)-para-tolylsulphinyl radical and does not reduce the group -COY.

16. Process according to either of Claims 14 and 15, characterized in that diisobutylaluminium hydride is used.

17. Process according to any one of Claims 14 to 16, characterized in that the reaction is conducted in an anhydrous medium, the reactants being introduced at a temperature below -20°C, and the medium is then allowed to return to room temperature.

18. Process according to any one of Claims 14 to 17, characterized in that the reaction is carried out in tetrahydrofuran and the reactants are introduced at a temperature of -50°C.

19. Process according to any one of Claims 14 to 18, characterized in that the reactants are introduced at a temperature in the region of -78°C.

20. Alkyl [3(X₃),5(X₅),S(Xs)]-3,5-dihydroxy-6-(para-tolylsulphinyl)hexanoate of formula (IV), 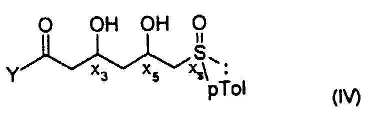 X₃ denoting the *R* or *S* configuration of the C₃ carbon, Y having the same meaning as in Claim 3, Xs denoting the *R* or *S* configuration of the sulphur, X₅ denoting the *R* or *S* configuration of the C₅ carbon, with X₅ ≠ Xs.

21. Alkyl [3(X₃),5(X₅),S(Xs)]-3,5-dihydroxy-6-(para-tolylsulphinyl)hexanoate according to Claim 20, characterized in that X₅ = X₃.

22. Process for preparing the products according to Claims 20 and 21, characterized in that the 3-carbonyl function of an alkyl [5(X₅),S(Xs)]-5-hydroxy-3-oxo-6-(para-tolylsulphinyl)hexanoate corresponding to the formula (III), 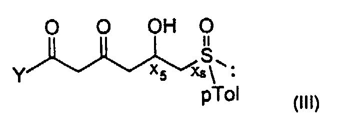 X₅ denoting the *R* or *S* configuration of the C₅ carbon, with X₅ ≠ Xs, Y having the same meaning as in Claim 3, Xs denoting the *R* or *S* configuration of the sulphur, is reduced by treatment with tetramethylammonium triacetoxyborohydride.

23. Alkyl [3(X₃),5(X₅),S(Xs)]-3,5-dihydroxy-6-(para-tolylsulphinyl)hexanoate according to Claim 20, characterized in that X₅ ≠ X₃.

24. Product according to Claim 23, characterized in that it is methyl [3(*R*),5(*S*),S(*R*)]-3,5-dihydroxy-6-(para-tolylsulphinyl)hexanoate.

25. Process for preparing the products according to Claims 23 and 24, characterized in that an alkyl [5(X₅),S(Xs)]-5-hydroxy-3-oxo-6-(para-tolylsulphinyl)hexanoate corresponding to the formula (III), 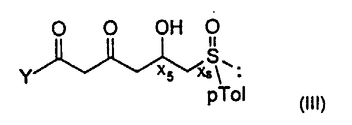 X₅ denoting the *R* or *S* configuration of the C₅ carbon, with X₅ ≠ Xs, Y having the same meaning as in Claim 3, Xs denoting the *R* or *S* configuration of the sulphur, is treated with sodium borohydride in the presence of a chelating agent.

26. Process according to Claim 25, characterized in that the chelating agent is a zinc, titanium or boron derivative.

27. Process according to Claim 26, characterized in that the chelating agent is chosen from tetraisopropyloxytitanium, zinc bromide and diethylmethoxyborane.

28. Process according to Claim 27, characterized in that the chelating agent is diethylmethoxyborane.

29. Process according to any one of Claims 25 to 28, characterized in that the reaction is conducted in an anhydrous medium.

30. Process according to any one of Claims 25 to 29, characterized in that the reactants are introduced at a temperature below 0°C.

31. Process according to any one of Claims 25 to 30, characterized in that the reaction is carried out in the presence of an excess of diethylmethoxyborane and sodium borohydride.

32. Process according to any one of Claims 25 to 31, characterized in that the reaction is carried out in tetrahydrofuran, introducing the reactants at a temperature below -20°C and preferably at -78°C.

33. Alkyl [3(X₃),5(X₅),S(Xs)]-3,5-dialkyloxy-6-(para-tolylsulphinyl)hexanoate of formula (V), 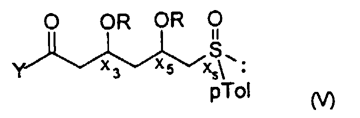 in which the two hydroxyl functions are protected by C1-C8 alkyl radicals or aryl or aralkyl radicals or trialkylsilyl radicals, or by a C₁-C₈ alkanediyl radical or a benzylidene radical, which radicals are designated R, Y having the same meaning as in Claim 3, Xs denoting the *R* or *S* configuration of the sulphur, X₃ having the same meaning as in Claim 20, X₅ denoting the *R* or *S* configuration of the C₅ carbon, with X₅ ≠ Xs.

34. Alkyl [3(X₃),5(X₅),S(Xs)]-3,5-dialkyloxy-6-(para-tolylsulphinyl)hexanoate according to Claim 33,
characterized in that R is chosen from methyl, benzyl, tert-butyldimethylsilyl, tert-butyldiphenylsilyl, isopropylidene and benzylidene radicals, the latter two radicals on their own enabling both hydroxyls to be protected simultaneously.

35. Alkyl [3(X₃),5(X₅),S(Xs)]-3,5-dialkyloxy-6-(para-tolylsulphinyl)hexanoate according to Claim 34, characterized in that R is an isopropylidene radical.

36. Product according to Claim 35, characterized in that it is methyl [3(*R*),5(*S*),S(*R*)]-3,5 -di(isopropylideneoxy)-6- (para-tolylsulphinyl)-hexanoate.

37. Process for preparing the product according to Claims 35 and 36, characterized in that the alkyl [3(X₃),5(X₅),S(Xs)]-3,5-dihydroxy-6-(para-tolylsulphinyl)hexanoate is treated with dimethoxypropane in the presence of catalytic amounts of para-toluenesulphonic acid in acetone.

38. Alkyl [3(X₃),5(X₅)]-6-acetoxy-3,5-dialkyloxy-6-(para-tolylthio)hexanoate of formula (VI) 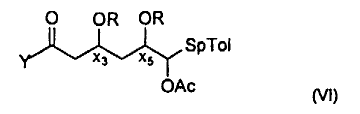 in which Y has the same meaning as in Claims 1 to 3, R has the same meaning as in Claim 33, X₃ has the same meaning as in Claim 20 and X₅ denotes the *R* or *S* configuration of the C₅ carbon.

39. Product according to Claim 38, characterized in that it is methyl [3(*R*),5(*S*)]-6-acetoxy-syn-3,5-di(isopropylideneoxy)-6-(para-tolylthio)-hexanoate.

40. Process for preparing an alkyl [3(X₃),5(X₅)]-6-hydroxy-3,5-dialkyloxyhexanoate corresponding to the formula (XI): 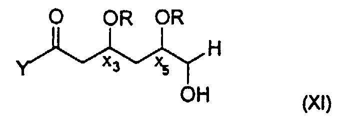 in which Y has the same meaning as in Claim 3, R has the same meaning as in Claim 33, X₃ has the same meaning as in Claim 20 and X₅ denotes the *R* or *S* configuration of the C₅ carbon, characterized in that the thioether function of a product according to either of Claims 38 and 39 is reduced and in that the acetate of this same product is hydrolysed.

41. Process according to Claim 40, characterized in that a treatment is carried out with Raney nickel in a protic solvent or in ethyl acetate, and in that the acetate is removed by treating the medium with a base.

42. Process according to Claim 41, characterized in that the acetate is removed by treatment with diisobutylaluminium carbonate or hydride.

43. Process for preparing an alkyl [3(X₃),5(X₅)]-6-oxo-3,5-dialkyloxyhexanoate corresponding to the formula (VII): 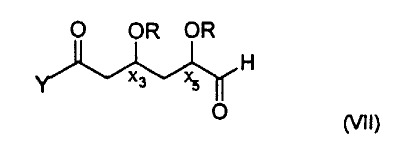 in which Y has the same meaning as in Claim 3, R has the same meaning as in Claim 33, X₃ has the same meaning as in Claim 20 and X₅ denotes the *R* or *S* configuration of the C₅ carbon, with X₅ ≠ Xs, characterized in that the product according to Claim 38 or 39 is treated with carbonate in an aqueous medium.

44. Process for preparing products corresponding to one of the formulae (Ia) and (Ib) 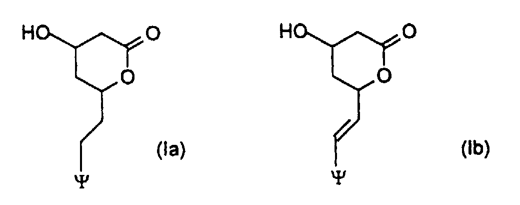 in which Ψ has the same meaning as below, characterized in that it comprises at least one step chosen from the following steps 1 to 8:
1) in the first step, the tri-anion of the alkyl 3,5-dioxohexanoate of formula (X) is reacted with (-)-or (+)-menthyl (X's)-p-toluenesulphinate (p-toluene designated pTol) to obtain the alkyl (Xs)-3,5-dioxo-6-(p-tolylsulphinyl)hexanoate of formula (II),
Y-CO-CH₂-CO-CH₂-CO-CH₃ (X) 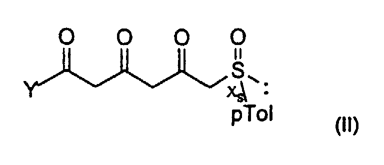
Xs and X's denoting the *R* or *S* configurations of the sulphur in the two molecules, with Xs ≠ X's, Y representing a saturated or unsaturated, linear or branched C₁-C₁₈ alkyloxy radical, an aryloxy radical or an aralkyloxy radical comprising not more than 18 carbon atoms, an imidazolide radical, a saturated or unsaturated, linear or branched C₁-C₁₈ alkyl sulphide radical, an aryl sulphide or C₁-C₂₀ aralkyl sulphide radical or a radical 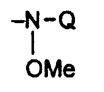 Q denoting a saturated or unsaturated, linear or branched C₁-C₁₈ alkyl radical,
2) in the second step, the 5-carbonyl function of the alkyl (Xs)-3,5-dioxo-6-(para-tolylsulphinyl)-hexanoate is reduced enantioselectively to alcohol to give the alkyl [5(X₅),S(Xs)]-5-hydroxy-3-oxo-6-(para-tolylsulphinyl)hexanoate of formula (III) below, X₅ denoting the configuration of the C₅ carbon, with X₅ ≠ Xs and Y having the same meaning as above, 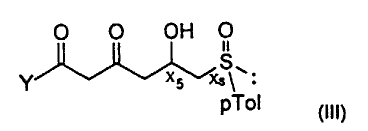
3) in the third step, the 3-carbonyl function of the alkyl [5(X₅),S(Xs)]-5-hydroxy-3-oxo-6-(para-tolylsulphinyl)hexanoate is reduced diastereo- selectively to obtain the alkyl [3(X₃),5(X₅),S(Xs)]-3,5-dihydroxy-6-(para-tolylsulphinyl)hexanoate of formula (IV) below, X₃ denoting the configuration of the C₃ carbon, choosing a method of reduction that gives X₅ = X₃ if an anti configuration is desired or a method of reduction that gives X₅ ≠ X₃ if a syn configuration is desired for the two alcohol functions, Y having the same meaning as above, 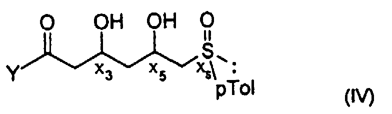
4) in the fourth step, the two hydroxyl functions are protected by C₁-C₈ alkyl radicals or aryl or aralkyl radicals or trialkylsilyl radicals, or by a C₁-C₈ alkanediyl radical or a benzylidene radical, which radicals will be designated R, to obtain an alkyl [3(X₃),5(X₅),S(Xs)]-3,5-dialkyloxy-6-(para-tolylsulphinyl)hexanoate of formula (V) below, Y having the same meaning as above, the said radicals not being displaced under the conditions of steps 5 and 6, 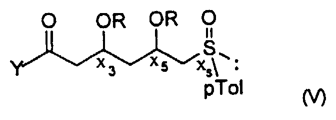
5) in the fifth step, the sulphoxide function is reduced to thioether by a Pummerer reaction to obtain an alkyl [3(X₃),5(X₅)]-6-acetoxy-3,5-dialkyloxy-6-(para-tolylthio)hexanoate of formula (VI) below, R and Y having the same meaning as above, 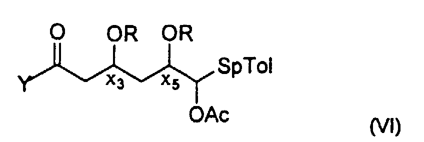
6) in the sixth step, the functions at C6 are converted to aldehyde to obtain the alkyl [3(X₃),5(X₅)]-6-oxo-3,5-dialkyloxyhexanoate corresponding to the formula (VII), Y and R having the same meaning as above, 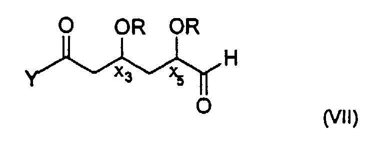
7) in the seventh step, the alkyl [3(X₃),5(X₅)]-6-oxo-3,5-dialkyloxyhexanoate of formula (VII) is condensed with a Wittig reagent Ph₃P=CHΨ, where Ψ represents a radical chosen from:
- saturated or unsaturated, linear, branched or cyclic C₁-C₄₀ alkyls, optionally interrupted by ether bridges and/or substituted with at least one substituent chosen from halogen, hydroxyl, C₁-C₂₀ alkoxy, C₁-C₂₀ acyloxy, primary, secondary or tertiary amino, nitro, thiol, carboxyl, amido, C₁-C₂₀ alkyl carboxylate, aryl carboxylate, C₁-C₂₀ aralkyl carboxylate, C₁-C₂₀ alkylamido, arylamido and C₁-C₂₀ aralkylamido groups, and from aryl radicals and saturated or unsaturated C₁-C₂₀ nitrogen, oxygen, phosphorus and sulphur heterocycles;
- aryls, optionally substituted with at least one substituent chosen from halogen, hydroxyl, C₁-C₂₀ alkoxy, C₁-C₂₀ acyloxy, amino, C₁-C₂₀ alkylamino and dialkylamino, nitro, thiol, carboxyl, amido, C₁-C₂₀ alkyl carboxylate, aryl carboxylate, C₁-C₂₀ aralkyl carboxylate, C₁-C₂₀ alkylamido, arylamido, C₁-C₂₀ aralkylamido and C₁-C₂₀ aryl groups, and from saturated or unsaturated, linear, branched or cyclic C₁-C₂₀ alkyl and aralkyl radicals and saturated or unsaturated C₁-C₂₀ nitrogen, oxygen, phosphorus and sulphur heterocycles;
- aralkyls, optionally substituted with at least one substituent chosen from halogen, hydroxyl, C₁-C₂₀ alkoxy, C₁-C₂₀ acyloxy, amino, C₁-C₂₀ alkylamino and dialkylamino, nitro, thiol, carboxylic acid, amido, C₁-C₂₀ alkyl carboxylate, aryl carboxylate, C₁-C₂₀ aralkyl carboxylate, C₁-C₂₀ alkylamido, arylamido, C₁-C₂₀ aralkylamido and C₁-C₂₀ aryl groups, and from saturated or unsaturated, linear, branched or cyclic C₁-C₂₀ alkyl and aralkyl radicals;
- nitrogen, oxygen or sulphur heterocycles, optionally substituted with at least one substituent chosen from halogen, hydroxyl, C₁-C₂₀ alkoxy, C₁-C₂₀ acyloxy, primary, secondary or tertiary amino, nitro, thiol, carboxylic acid, amido, C₁-C₂₀ alkyl carboxylate, aryl carboxylate, C₁-C₂₀ aralkyl carboxylate, C₁-C₂₀ alkylamido, arylamido and C₁-C₂₀ aralkylamido groups, and from aryl and aralkyl radicals and saturated or unsaturated C₁-C₂₀ nitrogen, oxygen, phosphorus and sulphur heterocycles
to give the product of formula (VIII), R and Y having the same meaning as above: 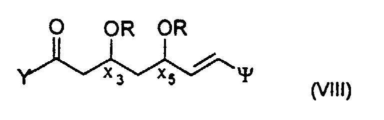
8) in the eighth step, the double bond is reduced to obtain the following product of formula (IX): 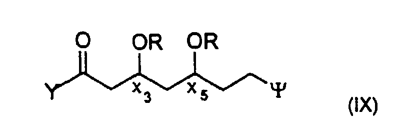
9) in the ninth and final step, the protective groups R are removed from the hydroxyls, and the ester -COY of the product of formula (VIII) or of the product of formula (IX) is hydrolysed to give the product of formula (Ib) or the product of formula (Ia), respectively.

## Patentansprüche

1. Alkyl-(Xs)-3,5-dioxo-6-p-tolylsulfinylhexanoate der Formel (II), 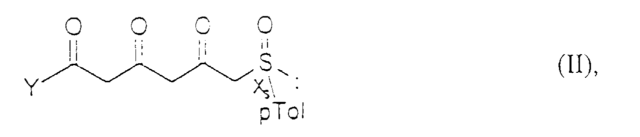 worin Xs die *R*- oder *S*-Konfiguration des Schwefels bezeichnet, gekennzeichnet durch Y = OCH₃.

2. Verbindung nach Anspruch 1, dadurch gekennzeichnet, daß es sich um das Methyl-(*R*)-3,5-dioxo-6-p-tolylsulfinylhexanoat handelt.

3. Verfahren zur Herstellung von Alkyl-(Xs)-3,5-dioxo-6-p-tolylsulfinylhexanoaten der Formel (II) 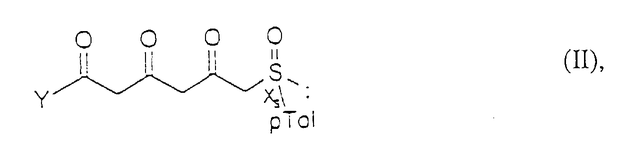 worin Xs und X's die Konfigurationen *R* oder *S* des Schwefelatoms bezeichnet und Y eine geradkettige oder verzweigte, gesättigte oder ungesättigte C₁₋₁₈-Alkyloxygruppe, eine Aryloxygruppe oder eine Aralkyloxygruppe mit 1 bis 18 Kohlenstoffatomen, eine Imidazolidgruppe, eine geradkettige oder verzweigte, gesättigte oder ungesättigte C₁₋₁₈-Alkylsulfidgruppe, Arylsulfid, C₁₋₁₈-Aralkylsulfid oder eine Gruppe 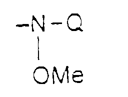 bedeutet, wobei Q eine geradkettige oder verzweigte, gesättigte oder ungesättigte C₁₋₁₈-Alkylgruppe ist, dadurch gekennzeichnet, daß das Trianion des Alkyl-3,5-dioxo-hexanoats der Formel (X)
Y-CO-CH₂-CO-CH₂-CO-CH₃ (X)
mit dem (X's)-p-Toluolsulfinat von (-) oder (+)Menthyl umgesetzt wird, wobei X's die *R*- oder *S*-Konfiguration des Schwefels bedeutet und X's ≠ Xs ist.

4. Verfahren nach Anspruch 3, dadurch gekennzeichnet, daß das Trianion des Alkyl-3,5-dioxo-hexanoats durch Umsetzung mit Natriumhydrid und *tert*.-Butyllithium oder *sek*.-Butyllithium in einem wasserfreien Medium bei niedriger Temperatur hergestellt wird.

5. Verfahren nach Anspruch 4, dadurch gekennzeichnet, daß das Trianion des Alkyl-3,5-dioxohexanoats durch Umsetzung mit etwa einem Äquivalent Natriumhydroxid und zwei Äquivalenten *tert*.-Butyllithium oder in Gegenwart von etwa einem Äquivalent Natriumhydrid und zwei Äquivalenten *sek*.-Butyllithium hergestellt wird.

6. Verfahren nach einem der Ansprüche 3 bis 5, dadurch gekennzeichnet, daß die Umsetzung in einem Lösungsmittel durchgeführt wird, das unter den Ethern ausgewählt ist.

7. Verfahren nach Anspruch 5, dadurch gekennzeichnet, daß die Reaktion in Tetrahydrofuran durchgeführt wird.

8. Verfahren nach einem der Ansprüche 3 bis 7, dadurch gekennzeichnet, daß die Umsetzung bei einer Temperatur von -10 bis +10 °C durchgeführt wird.

9. Verfahren nach Anspruch 8, dadurch gekennzeichnet, daß die Umsetzung bei einer Temperatur von etwa 0 °C durchgeführt wird.

10. Verfahren nach einem der Ansprüche 3 bis 9, dadurch gekennzeichnet, daß die Umsetzung mit einer Menge von Menthyl-p-Toluolsulfinat durchgeführt wird, die etwa 50 % der Molzahl von Alkyl-3,5-dioxyhexanoat entspricht.

11. Verfahren nach einem der Ansprüche 3 bis 10, dadurch gekennzeichnet, daß das (*S*)-p-Toluolsulfinat von (-)Menthyl oder das (*R*)-p-Toluolsulfinat von (+)Menthyl verwendet wird.

12. Verfahren nach Anspruch 10, dadurch gekennzeichnet, daß das (*S*)-p-Toluolsulfinat von (-)Menthyl verwendet wird.

13. Methyl-[5(*S*), S(*R*)]-5-hydroxy-3-oxo-6-paratolylsulfinalhexanoat.

14. Verfahren zur Herstellung von Alkyl-[5(X₅), S(X_{S})]-5-hydroxy-3-oxo-6-para-tolylsulfinylhexanoaten der Formel (III) 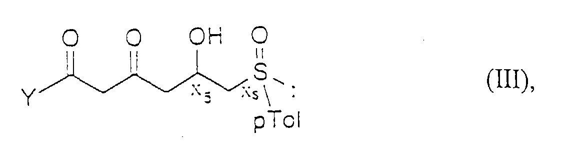 worin X₅ die *R-* oder *S*-Konfiguration des Kohlenstoffs C₅ mit X₅ ≠ Xs bedeutet, Y die oben in Anspruch 3 angegebenen Bedeutungen aufweist, Xs die *R*- oder *S*-Konfiguration des Schwefels bedeutet, dadurch gekennzeichnet, daß die C5-Carbonylgruppe des Alkyl-(Xs)-3,5-dioxo-6-paratolylsulfinylhexanoats der Formel (II) enantioselektiv zur Alkoholgruppe reduziert wird: 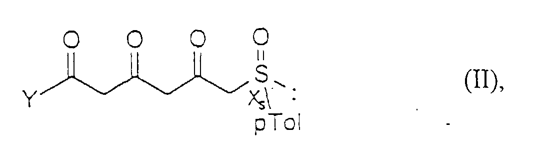 worin Xs die *R*- oder *S*-Konfiguration des Schwefels bedeutet.

15. Verfahren nach Anspruch 14, dadurch gekennzeichnet, daß die Reduktion mit einem Hydrid durchgeführt wird, das die stereochemische Induktion durch die (Xs)-p-Tolylsulfinylgruppe nicht verändert und die Gruppe -COY nicht reduziert.

16. Verfahren nach einem der Ansprüche 14 und 15, dadurch gekennzeichnet, daß Diisobutylaluminiumhydrid verwendet wird.

17. Verfahren nach einem der Ansprüche 14 bis 16, dadurch gekennzeichnet, daß die Umsetzung in einem wasserfreien Medium durchgeführt wird, wobei die Reaktanten bei einer Temperatur von -20 °C zugegeben werden und das Medium dann auf Raumtemperatur zurückkommen gelassen wird.

18. Verfahren nach einem der Ansprüche 14 bis 17, dadurch gekennzeichnet, daß die Umsetzung in Tetrahydrofuran durchgeführt wird und die Reaktanten bei einer Temperatur von -50 °C zugegeben werden.

19. Verfahren nach einem der Ansprüche 14 bis 18, dadurch gekennzeichnet, daß die Reaktanten bei einer Temperatur von etwa -78 °C zugegeben werden.

20. Alkyl-[3(X₃), 5(X₅), S(Xs)]-3,5-dihydroxy-6-paratolylsulfinylhexanoat der Formel (IV) 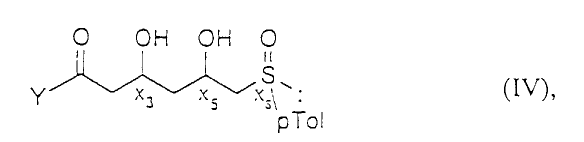 worin X₃ die *R*- oder *S*-Konfiguration des Kohlenstoffs C₃ bedeutet, Y die oben in Anspruch 3 angegebenen Bedeutungen aufweist, Xs die *R-* oder *S*-Konfiguration des Schwefels bedeutet und X₅ die *R-* oder *S*-Konfiguration des Kohlenstoffs C₅ mit X₅ ≠ Xs bedeutet.

21. Alkyl-[3(X₃), 5(X₅), S(Xs)]-3,5-dihydroxy-6-paratolylsulfinylhexanoat nach Anspruch 20, gekennzeichnet durch X₅ = X₃.

22. Verfahren zur Herstellung von Verbindungen nach den Ansprüchen 20 und 21, dadurch gekennzeichnet, daß die C3-Carbonylgruppe des Alkyl-[5(X₅), S(Xs)]-5-hydroxy-3-oxo-6-paratolylsulfinylhexanoats der Formel (III): 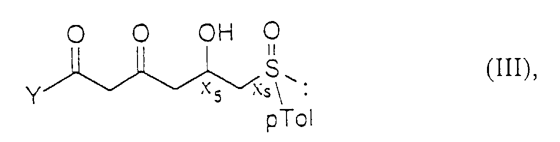 worin X₅ die *R*- oder *S*-Konfiguration des Kohlenstoffs C₅ mit X₅ ≠ Xs bedeutet, Y die in Anspruch 3 angegebenen Bedeutungen aufweist und Xs die *R*- oder *S*-Konfiguration des Schwefels bedeutet durch Umsetzung mit Tetramethylammoniumtriacetoxyborhydrid reduziert wird.

23. Alkyl-[3(X₃), 5(X₅), S(Xs)]-3,5-dihydroxy-6-paratolylsulfinylhexanoat nach Anspruch 20, gekennzeichnet durch X₅ ≠ X₃.

24. Verbindungen nach Anspruch 23, dadurch gekennzeichnet, daß es sich um das Methyl-[3(*R*), 5(*S*), S(*R*)]-3,5-dihydroxy-6-paratolylsulfinylhexanoat handelt.

25. Verfahren zur Herstellung von Verbindungen nach den Ansprüchen 23 und 24, dadurch gekennzeichnet, daß ein Alkyl-[5(X₅), S(Xs)]-5-hydroxy-3-oxo-6-paratolylsulfinylhexanoat der Formel (III) 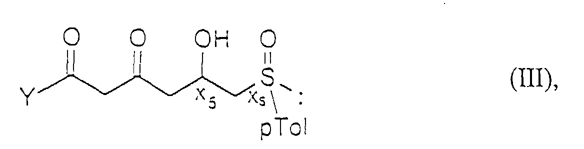 worin X₅ die *R*- oder *S*-Konfiguration des Kohlenstoffs X₅ mit X₅ ≠ Xs bedeutet, Y die in Anspruch 3 angegebenen Bedeutungen aufweist und Xs die *R*- oder *S*-Konfiguration des Schwefels bedeutet, mit Natriumborhydrid in Gegenwart eines Chelatbildners umgesetzt wird.

26. Verfahren nach Anspruch 25, dadurch gekennzeichnet, daß der Chelatbildner ein Derivat von Zink, Titan oder Bor ist.

27. Verfahren nach Anspruch 26, dadurch gekennzeichnet, daß der Chelatbildner unter Tetraisopropyloxytitan, Zinkbromid und Diethylmethoxyboran ausgewählt ist.

28. Verfahren nach Anspruch 27, dadurch gekennzeichnet, daß der Chelatbildner das Diethylmethoxyboran ist.

29. Verfahren nach einem der Ansprüche 25 bis 28, dadurch gekennzeichnet, daß die Umsetzung in einem wasserfreien Medium durchgeführt wird.

30. Verfahren nach einem der Ansprüche 25 bis 29, dadurch gekennzeichnet, daß die Reaktanten bei einer Temperatur unter 0 °C zugegeben werden.

31. Verfahren nach einem der Ansprüche 25 bis 30, dadurch gekennzeichnet, daß die Umsetzung in Gegenwart eines Überschusses von Diethylmethoxyboran und Natriumborhydrid durchgeführt wird.

32. Verfahren nach einem der Ansprüche 25 bis 31, dadurch gekennzeichnet, daß die Umsetzung in Tetrahydrofuran durchgeführt wird, wobei die Reaktanten bei einer Temperatur unter -20 °C und vorzugsweise bei -78 °C zugegeben werden.

33. Alkyl-[3(X₃), 5(X₅), S(Xs)]-3,5-dialkyloxy-6-paratolylsulfinylhexanoat der Formel (V) 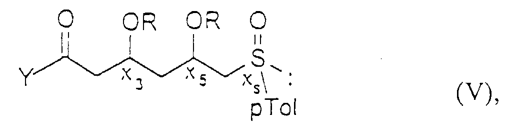 worin die beiden Hydroxygruppen mit X₁₋₈-Alkyl, Aryl, Aralkyl, Trialkylsilyl, C₁₋₈-Alkandiyl oder Benzyliden geschützt sind, wobei die Gruppen als R bezeichnet werden, Y die in Anspruch 3 angegebenen Bedeutungen aufweist, Xs die *R*- oder *S*-Konfiguration des Schwefels bedeutet, X₃ die in Anspruch 20 angegebenen Bedeutungen aufweist und X₅ die *R*- oder *S*-Konfiguration des Kohlenstoffatoms C₅ mit X₅ ≠ Xs bedeutet.

34. Alkyl-[3(X₃), 5(X₅), S(Xs)]-3,5-dialkyloxy-6-paratolylsulfinylhexanoat nach Anspruch 33, dadurch gekennzeichnet, daß R ausgewählt ist unter den Gruppen Methyl, Benzyl, *tert*.-Butyldimethylsilyl, *tert*.-Butyldiphenylsilyl, Isopropyliden und Benzyliden, wobei die beiden letzten Gruppen alleine gleichzeitig beide Hydroxygruppen schützen können.

35. Alkyl-[3(X₃), 5(X₅), S(Xs)]-3,5-dialkyloxy-6-paratolylsulfinylhexanoat nach Anspruch 34, dadurch gekennzeichnet, daß R die Diisopropylidengruppe ist.

36. Verbindung nach Anspruch 35, dadurch gekennzeichnet, daß es sich um das Methyl-[3(*R*), 5(*S*), S(*R*)]-3,5-dioxyisopropyliden-6-paratolylsulfinylhexanoat handelt.

37. Verfahren zur Herstellung der Verbindungen nach den Ansprüchen 35 und 36, dadurch gekennzeichnet, daß das Alkyl-[3(X₃), 5(X₅), S(Xs)]-3,5-dihydroxy-6-paratolylsulfinylhexanoat in Gegenwart von katalytischen Mengen von p-Toluolsulfonsäure in Aceton mit Dimethoxypropan umgesetzt wird.

38. Alkyl-[3(X₃), 5(X₅)]-6-acetoxy-3,5-dialkyloxy-6-paratolylthiohexanoate der Formel (VI) 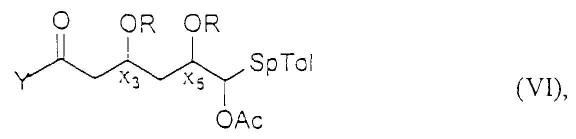 worin Y die in den Ansprüchen 1 bis 3 angegebenen Bedeutungen aufweist, R die in Anspruch 33 angegebenen Bedeutungen aufweist, X₃ die in Anspruch 20 angegebenen Bedeutungen aufweist und X₅ die *R*- oder *S*-Konfiguration des Kohlenstoffatoms C₅ bedeutet.

39. Verbindungen nach Anspruch 38, dadurch gekennzeichnet, daß es sich um das Methyl-[3(*R*), 5(*S*)]-6-acetoxy-syn-3,5-dioxyisopropyliden-6-paratolylthiohexanoat handelt.

40. Verfahren zur Herstellung von Alkyl-[3(X₃), 5(X₅)]-6-hydroxy-3,5-dialkyloxyhexanoaten der Formel (IX) 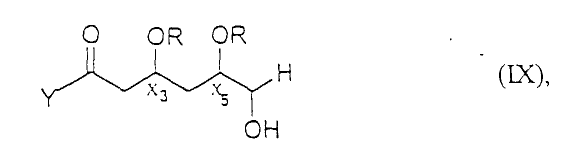 worin Y die in Anspruch 3 angegebene Bedeutung aufweist, R die in Anspruch 33 angegebene Bedeutung aufweist, X₃ die in Anspruch 20 angegebene Bedeutung aufweist und X₅ die *R*- oder *S*-Konfiguration des Kohlenstoffatoms C₅ bedeutet, dadurch gekennzeichnet, daß die Thioethergruppe der Verbindungen nach einem der Ansprüche 38 bis 39 reduziert und das Acetat hydrolysiert wird.

41. Verfahren nach Anspruch 40, dadurch gekennzeichnet, daß eine Umsetzung mit Raney-Nickel in einem protischen Lösungsmittel oder in Ethylacetat durchgeführt wird und das Acetat durch Behandlung des Mediums mit einer Base entfernt wird.

42. Verfahren nach Anspruch 41, dadurch gekennzeichnet, daß das Acetat durch eine Behandlung mit dem Carbonat oder Hydrid von Diisobutylaluminium entfernt wird.

43. Verfahren zur Herstellung von Alkyl-[3(X₃), 5(X₅)]-6-oxo-3,5-dialkyloxyhexanoaten der Formel (VII) 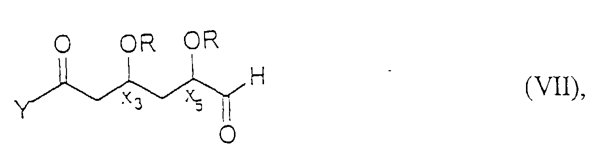 worin Y die in Anspruch 3 angegebenen Bedeutungen aufweist, R die in Anspruch 33 angegebenen Bedeutungen aufweist, X₃ die in Anspruch 20 angegebenen Bedeutungen aufweist und X₅ die *R*- oder *S*-Konfiguration des Kohlenstoffatoms C₅ mit X₅ ≠ Xs bedeutet, dadurch gekennzeichnet, daß die Verbindung nach Anspruch 38 oder 39 in einem wäßrigen Medium mit einem Carbonat behandelt wird.

44. Verfahren zur Herstellung von Verbindungen, die einer der Formeln (Ia) und (Ib) entsprechen 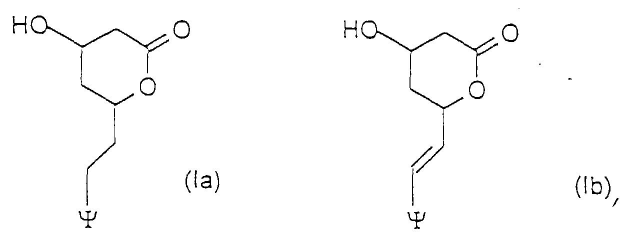 worin Ψ die darunter angegebenen Bedeutungen aufweist, dadurch gekennzeichnet, daß es mindestens einen Schritt umfaßt, der unter den folgenden Schritten 1 bis 8 ausgewählt ist:
1) in einem ersten Schritt wird das Trianion von Alkyl-3,5-dioxohexanoat der Formel (X) mit (X's)-p-Toluol (im folgenden als pTol bezeichnet)-sulfinat von (-) oder (+)Menthyl umgesetzt, wodurch das Alkyl (Xs)-3,5-dioxo-6-p-tolylsulfinylhexanoat der Formel (II) hergestellt wird:
Y-CO-CH₂-CO-CH₂-CO-CH₃ (X) 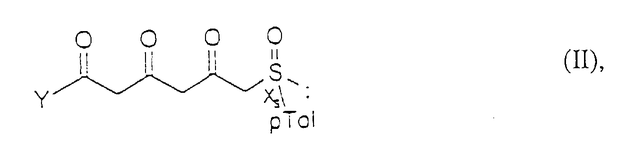
worin Xs und X's die Konfigurationen *R* oder *S* des Schwefelatoms in den beiden Molekülen bezeichnen, mit Xs ≠ X's; Y eine geradkettige oder verzweigte, gesättigte oder ungesättigte C₁₋₁₈-Alkyloxygruppe, eine Aryloxygruppe oder eine Aralkyloxygruppe mit höchstens 18 Kohlenstoffatomen, eine Imidazolidgruppe, eine geradkettige oder verzweigte, gesättigte oder ungesättigte C₁₋₁₈-Alkylsulfidgruppe, Arylsulfid, C₁₋₁₈-Aralkylsulfid oder eine Gruppe 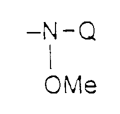 bedeutet, wobei Q eine geradkettige oder verzweigte, gesättigte oder ungesättigte C₁₋₁₈-Alkylgruppe ist;
2) in einem zweiten Schritt wird die C5-Carbonylgruppe des Alkyl-(Xs)-3,5-dioxo-6-paratolylsulfinylhexanoats enantioselektiv zu einer Alkoholgruppe reduziert, wodurch das Alkyl-[5(X₅), S(Xs)]-5-hydroxy-3-oxo-6-paratolylsulfinylhexanoat der nachfolgend angegebenen Formel (III) gebildet wird, wobei X₅ die Konfiguration des Kohlenstoffatoms C₅ mit X₅ ≠ Xs bedeutet und Y die oben angegebenen Bedeutungen aufweist 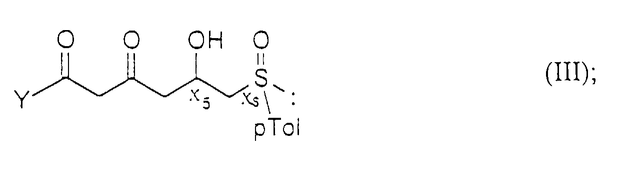
3) in einem dritten wird Schritt die C3-Carbonylgruppe des Alkyl-[5(X₅). S(Xs)]-5-hydroxy-3-oxo-6-paratolylsulfinylhexanoats diastereoselektiv reduziert, wodurch das Alkyl-[3(X₃), 5(X₅), S(Xs)]-3,5dihydroxy-6-paratolylsulfinylhexanoat der folgenden Formel (IV) gebildet wird, wobei X₃ die Konfiguration des Kohlenstoffatoms C₃ bedeutet, wobei ein Reduktionsverfahren gewählt wird, das X₅ = X₃ ergibt, wenn eine Antikonfiguration gebildet werden soll, oder ein Reduktionsverfahren gewählt wird, das X₅ ≠ X₃ ergibt, wenn eine Synkonfiguration der beiden Alkoholgruppen gebildet werden soll, wobei Y die oben angegebenen Bedeutungen aufweist 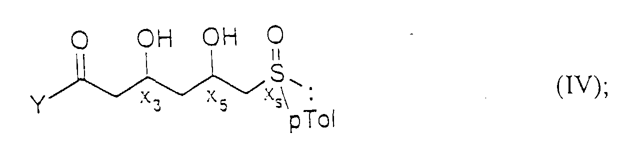
4) in einem vierten Schritt werden die beiden Hydroxygruppen mit C₁₋₈-Alkyl, Aryl, Aralkyl, Trialkylsilyl, C₁₋₈-Alkandiyl oder Benzyliden geschützt, wobei diese Gruppen als R bezeichnet werden, wodurch ein Alkyl-[3(X₃), 5(X₅), S(Xs)]-3,5-dialkyloxy-6-paratolylsulfinylhexanoat der Formel (V) hergestellt wird, wobei Y die oben angegebenen Bedeutungen aufweist und wobei diese Gruppen unter den Bedingungen der Schritte 5 und 6 nicht entfernt werden 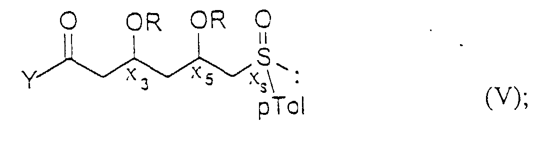
5) in einem fünften wird Schritt wird die Sulfoxidgruppe über eine Pummerer-Umlagerung in eine Thioethergruppe umgewandelt, wodurch ein Alkyl-[3(X₃), 5(X₅)]-6-acetoxy-3,5-dialkyloxy-6-paratolylthiohexanoat der folgenden Formel (VI) hergestellt wird, worin R und Y die oben angegebenen Bedeutungen aufweisen: 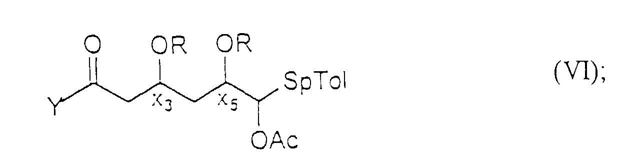
6) in einem sechsten Schritt wird die Gruppe in C₆-Stellung in einen Aldehyden umgewandelt, wodurch ein Alkyl-[3(X₃), 5(X₅)]-6-oxo-3,5-dialkyloxyhexanoat der Formel (VII) gebildet, wobei Y und R die oben angegebenen Bedeutungen aufweisen: 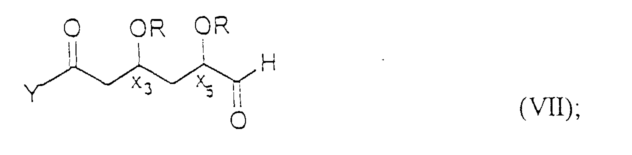
7) in einem siebten Schritt wird das Alkyl-[3(X₃), 5(X₅)]-6-oxo-3,5-dialkyloxyhexanoat der Formel (VII) mit einem Wittig-Reagenz Ph₃P=CHψ kondensiert, wobei ψ eine Gruppe bedeutet, die ausgewählt ist unter:
- den geradkettigen, verzweigten oder cyclischen, gesättigten oder ungesättigten C₁₋₄₀-Alkylgruppen, die gegebenenfalls durch Etherbrücken unterbrochen sind und/oder gegebenenfalls mit einem oder mehreren Substituenten substituiert sind, die unter Halogen, Hydroxy, C₁₋₂₀-Alkoxy, C₁₋₂₀-Acyloxy, primären, sekundären oder tertiären Aminogruppen, Nitro, Thiol, Carboxy, Amido, C₁₋₂₀-Alkylcarboxylat, Arylcarboxylat, C₁₋₂₀-Aralkyl-carboxylat, C₁ ₂₀-Alkylamido, Arylamido, C₁₋₂₀-Aralkylamido und unter den Arylgruppen und Stickstoff-, Sauerstoff-, Phosphor- und Schwefelheterocyclen mit 1 bis 20 Kohlenstoffatomen, die gesättigt oder ungesättigt sind, ausgewählt sind;
- den Arylgruppen, die gegebenenfalls mit einem oder mehreren Substituenten substituiert sind, die unter Halogen, Hydroxy, C₁₋₂₀-Alkoxy, C₁₋₂₀-Acyloxy, Amino, C₁₋₂₀-Alkylamino und C₁₋₂₀-Dialkylamino, Nitro, Thiol, Carboxy, Amido, C₁₋₂₀-Alkylcarboxylat, Arylcarboxylat, C₁₋₂₀-Aralkylcarboxylat, C₁₋₂₀-Alkylamido, Arylamido, C₁₋₂₀-Aralkylamido, C₁₋₂₀-Aryl und unter den gesättigten oder ungesättigten, geradkettigen, verzweigten oder cyclischen Alkyl- und Aralkylgruppen mit 1 bis 20 Kohlenstoffatomen und gesättigten oder ungesättigten Stickstoff-, Sauerstoff-, Phosphor- und Schwefelheterocyclen ausgewählt sind;
- den Aralkylgruppen, die gegebenenfalls mit einem oder mehreren Substituenten substituiert sind, die unter Halogen, Hydroxy, C₁₋₂₀-Alkoxy, C₁₋₂₀-Acyloxy, Amino, Alkylaniino und Dialkylamino mit 1 bis 20 Kohlenstoffatomen, Nitro, Thiol, Carboxy, Amido, C₁₋₂₀-Alkylcarboxylat, Arylcarboxylat, C₁₋₂₀-Aralkylcarboxylat, C₁₋₂₀-Alkylamido, Arylamido, C₁₋₂₀-Aralkylamido, C₁₋₂₀-Aryl und unter den gesättigten oder ungesättigten, geradkettigen, verzweigten oder cyclischen Alkyl- und Aralkylgruppen mit 1 bis 20 Kohlenstoffatomen ausgewählt sind; und
- den Stickstoff-, Sauerstoff-, Phosphor- oder Schwefelheterocyclen, die gegebenenfalls mit einem oder mehreren Substituenten substituiert sind, die unter Halogen, Hydroxy, C₁₋₂₀-Alkoxy, C₁₋₂₀-Acyloxy, primären, sekundären oder tertiären Aminogruppen, Nitro, Thiol, Carboxy, Amido, C₁₋₂₀-Alkylcarboxylat, Arylcarboxylat, C₁₋₂₀-Aralkylcarboxylat, C₁₋₂₀-Alkylamido, Arylamido, C₁₋₂₀-Aralkylamido und unter den Gruppen Aryl, Aralkyl und Stickstoff-, Sauerstoff-, Phosphor- und Schwefelheterocyclen mit 1 bis 20 Kohlenstoffatomen, die gesättigt oder ungesättigt sind, ausgewählt sind, wodurch das Produkt der Formel (VIII) gebildet wird, worin R und Y die oben angegebenen Bedeutungen aufweisen: 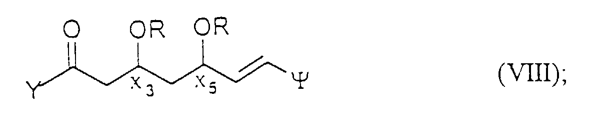
8) in einem achten Schritt wird die Doppelbindung reduziert, wodurch erhalten wird: 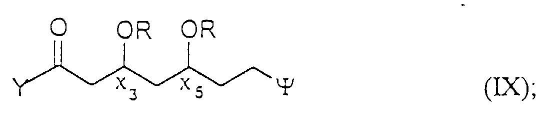 und
9) in einem neunten und letzten Schritt werden die Schutzgruppen der Hydroxygruppen entfernt und der Ester -COY der Verbindungen der Formel (VIII) und (IX) hydrolysiert, wodurch die Verbindung der Formel (Ia) bzw. (Ib) entsteht.
